# EUROPEAN PATENT SPECIFICATION

(11) **EP 1 773 401 B1**
(45) Date of publication and mention of the grant of the patent: **02.01.2013**
(21) Application number: 05767672.8
(22) Date of filing: 21.06.2005
(51) Int. Cl.: A61K 48/00, C12N 15/86

(54) **MEDICAL SYSTEMS AND METHODS FOR DELIVERING COMPOSITIONS TO CELLS**
MEDIZINISCHE SYSTEME UND VERFAHREN ZUR ABGABE VON ZUSAMMENSETZUNGEN AN ZELLEN
SYSTEMES ET PROCEDES MEDICAUX PERMETTANT L'ADMINISTRATION DE COMPOSITIONS A DES CELLULES

(30) Priority: 21.06.2004 US 581730 P
(43) Date of publication of application: 18.04.2007
(73) Proprietor: Medtronic, Inc., Minneapolis, MN 55432-5604 (US)
(72) Inventor: KAEMMERER, William, F., Edina, MN 55435 (US); BURRIGHT, Eric, N., Eagan, MN 55123 (US); TENBROEK, Erica, M., Roseville, MN 55113 (US); BLUM, Janelle, L., St. Paul, MN 55117 (US); KAYTOR, Michael, D., Maplewood, MN 55117 (US)
(74) Representative: Hughes, Andrea Michelle
(86) International application number: PCT/US2005/022156
(87) International publication number: WO 2006/002283

(56) References cited:
- WO-A-01/70276
- WO-A-98/46273
- WO-A-2004/047872
- US-A1- 2002 076 394
- US-B1- 6 468 524
- FU HAIYAN ET AL: "Self-complementary adeno-associated virus serotype 2 vector: global distribution and broad dispersion of AAV-mediated transgene expression in mouse brain." MOLECULAR THERAPY : THE JOURNAL OF THE AMERICAN SOCIETY OF GENE THERAPY. DEC 2003, vol. 8, no. 6, December 2003 (2003-12), pages 911-917, XP002350611 ISSN: 1525-0016
- DAVIDSON B L ET AL: "Molecular medicine for the brain: silencing of disease genes with RNA interference" LANCET NEUROLOGY, LANCET PUBLISHING GROUP, LONDON, GB, vol. 3, no. 3, March 2004 (2004-03), pages 145-149, XP004808832 ISSN: 1474-4422
- BOILLÉE S ET AL: "Gene therapy for ALS delivers" TRENDS IN NEUROSCIENCES 2004 UNITED KINGDOM, vol. 27, no. 5, 2004, pages 235-238, XP002350612 ISSN: 0166-2236
- XIA H ET AL: "siRNA-mediated gene silencing in vitro and in vivo" NATURE BIOTECHNOLOGY, NATURE PUBLISHING, US, vol. 20, no. 10, October 2002 (2002-10), pages 1006-1010, XP002251054 ISSN: 1087-0156

## Description

### BACKGROUND

The delivery of biologically active agents to the brain is an important and challenging aspect of treating a variety of neurological disorders. For treatment of some neurological disorders, it is desirable to deliver a biologically active agent (e.g., a therapeutic agent) to the brain that will cause brain cells to express DNA, for example, a missing gene (i.e., gene therapy), and/or RNA, for example, a small interfering RNA (siRNA).

Some approaches to gene therapy for neurological disorders involve surgical delivery of non-viral or viral vectors directly into the brain tissue, which is generally necessary since non-viral and viral vectors normally do not cross the blood-brain barrier (BBB). These approaches are limited by difficulty in achieving sufficient distribution and diffusion of the vector into the targeted areas of the brain, and by the potential for viral vectors to produce an immune reaction in the patient. One approach for achieving enhanced diffusion of vectors into the brain tissue is to use the technique of "convection enhanced delivery," whereby the non-viral or viral vectors are administered at a low flow rate over a long period of time with a pump providing pressure and flow volume to enhance the distribution of the vector into the tissue. While convection enhanced delivery has been shown to yield delivery of molecules and virus particles to substantial three-dimensional regions of rodent and primate brains, scale-up of this delivery approach to the three-dimensional volume of the human brain remains a technical challenge. Effective treatment of certain neurological diseases (e.g., Alzheimer's disease) using a gene or protein delivery or suppression therapy will most likely require delivery of the biologically active agents to most of the human cerebrum. In other neurological disorders, such as Parkinson's disease and Huntington's disease, even though there are circumscribed regions of the brain anatomy that are especially affected by the disease process, for example, the substantia nigra or striatum (caudate and putamen) and result in cardinal symptoms of the diseases (e.g., dyskinesias, rigidity, etc.), patients will likely benefit further from treatment of broader regions of the brain, in which the disease process causes additional symptoms (e.g., depression and cognitive deficits).

An approach of using viral vectors to deliver genes or gene suppressing agents to the brain tissue using stereotactic neurosurgery including, for example, the use of adeno-associated virus (AAV) to deliver gene therapy to the subthalamic nucleus, has shown considerable promise. However, the usefulness of stereotactic neurosurgery to deliver a viral vector carrying a gene or protein suppression therapy can be limited by one or more of the following factors. Stereotactic neurosurgery always involves a low level of surgical risk including, for example, accidental perforation of a blood vessel, which can result in cerebral hemorrhage and death. Dispersion of a viral vector to large regions of brain tissue, even using convection enhanced delivery and optimal vectors, catheter designs, and surgical technique, is likely to be limited relative to what can be attained using the blood stream as the distribution system. Manufacturing of viral particles (e.g., capsid plus DNA payload) in sufficient quantities for therapeutic use, while feasible, is costly relative to production of DNA alone. Viral particles (i.e., the capsid proteins) might be immunogenic, causing adverse reactions in sensitized individuals. While the immune response to some viruses (e.g., AAV) when administered to the brain appears minimal, it remains a potential limitation particularly for repeated therapy administrations.

It would be advantageous to administer a biologically active agent by a route that is no more invasive than a simple intravenous injection. With this approach, a biologically active agent could be delivered through the BBB by targeting the biologically active agent to the brain via endogenous BBB transport systems. Expression of a DNA or RNA in the brain requires that the biologically active agent that is injected into the blood is transported not only across the BBB by, for example, receptor-mediated transcytosis (RMT), but also across the brain cell membrane (BCM) by, for example, receptor-mediated endocytosis (RME) into the target cell in the brain. In addition, using endogenous BBB transport systems to target biologically active agents non-invasively to the brain also requires the development of a suitable formulation of the biologically active agent that is stable in the bloodstream.

An effective method for delivering gene therapy to the entire primate brain using compositions that carry plasmid DNA or antisense RNA across the blood brain barrier and into brain cells was recently disclosed in U.S. Patent No. 6,372,250 (Pardridge). The reported ability of this method to deliver plasmid DNA to the entire primate brain constitutes an impressive technical breakthrough. However, therapeutic use of the disclosed method may be limited by one or more of the factors listed herein below. Gene expression from a plasmid or RNA is generally temporary (e.g., limited to a period of days or weeks). Intravenous delivery of the disclosed compositions can result in unintended treatment of all bodily organs, potentially resulting in adverse side-effects. Finally, intravenous delivery can result in a loss of dosing as the dose intended for the brain is delivered to other parts of the body.

Thus, new compositions and methods for delivering biologically active agents to the brain are needed.

### SUMMARY OF THE INVENTION

In one aspect, the present invention provides a medical system for delivering single stranded DNA across a blood-brain barrier, the system comprising: a neurovascular catheter having a distal end to be positioned in a blood vessel supplying a patient's brain; composition comprising; an artificial adeno-associated virus (AAV) vector comprising single stranded DNA encoding a biologically active agent; and a component to deliver at least the single stranded DNA across the blood-brain barrier, wherein: a) said single stranded DNA encoding the biologically active agent is SEQ ID NO: 2; or b) said AAV is selected from SEQ ID NOs: 8-11; and a means for delivering said composition to the catheter.

In another embodiment, the system includes a neurovascular catheter having a distal end to be positioned in a blood vessel supplying a patient's brain; and a means for delivering to the catheter a composition including a receptor-specific liposome, wherein the receptor-specific liposome includes: a liposome having an exterior surface and an internal compartment; an artificial adeno-associated virus (AAV) vector located within the internal compartment of the liposome, wherein the AAV vector includes the single stranded DNA encoding a biologically active agent; one or more blood-brain barrier and brain cell membrane targeting agents; and one or more conjugation agents wherein each targeting agent is connected to the exterior surface of the liposome via at least one of the conjugation agents.

Also described herein is a method for delivering DNA across a blood-brain barrier for expression in the brain. The method includes administering to a patient a composition including a receptor-specific liposome, wherein the receptor-specific liposome includes: a liposome having an exterior surface and an internal compartment; an artificial adeno-associated virus (AAV) vector located within the internal compartment of the liposome, wherein the AAV vector includes DNA encoding a biologically active agent; one or more blood-brain barrier and brain cell membrane targeting agents; and one or more conjugation agents wherein each targeting agent is connected to the exterior surface of the liposome via at least one of the conjugation agents.

Also described herein is a method for delivering DNA to a cell. The method includes administering to a patient a composition including a receptor-specific nanocontainer, wherein the receptor-specific nanocontainer includes: a nanocontainer having an exterior surface and an internal compartment; an artificial adeno-associated virus (AAV) vector located within the internal compartment of the nanocontainer, wherein the AAV vector includes DNA encoding a biologically active agent; one or more receptor specific targeting agents that target the receptor located on the cell; and one or more conjugation agents wherein each targeting agent is connected to the exterior surface of the nanocontainer via at least one of the conjugation agents.

Also described herein is a method for delivering DNA across a blood-brain barrier for expression in the brain. The method includes administering to a patient a composition including: an artificial adeno-associated virus (AAV) vector including DNA encoding a biologically active agent; and a component to deliver at least the DNA across the blood-brain barrier.

Also described herein is a method of treating a neurodegenerative disorder caused by a pathogenic protein.

In one example, the method includes: providing a neurovascular catheter having a distal end positioned in a blood vessel supplying a patient's brain; and delivering to the catheter a composition including: an artificial adeno-associated virus (AAV) vector including DNA encoding a biologically active agent; and a component to deliver at least the DNA across the blood-brain barrier.

In another example, the method includes: providing a neurovascular catheter having a distal end positioned in a blood vessel supplying a patient's brain; and delivering to the catheter a composition including a receptor-specific liposome and a pharmaceutically acceptable carrier for the receptor-specific liposome, wherein the receptor-specific liposome includes: a liposome having an exterior surface and an internal compartment; an artificial adeno-associated virus (AAV) vector located within the internal compartment of the liposome, wherein the AAV vector includes DNA encoding a biologically active agent; one or more blood-brain barrier and brain cell membrane targeting agents; and one or more conjugation agents wherein each targeting agent is connected to the exterior surface of the liposome via at least one of the conjugation agents.

Also described herein is a method of treating a neurological disease caused by the absence of a protein.

In one example, the method includes: providing a neurovascular catheter having a distal end positioned in a blood vessel supplying a patient's brain; and delivering to the catheter a composition including: an artificial adeno-associated virus (AAV) vector including DNA encoding a biologically active agent; and a component to deliver at least the DNA across the blood-brain barrier.

In another example, the method includes: providing a neurovascular catheter having a distal end positioned in a blood vessel supplying a patient's brain; and delivering to the catheter a composition including a receptor-specific liposome and a pharmaceutically acceptable carrier for the receptor-specific liposome, wherein the receptor-specific liposome includes: a liposome having an exterior surface and an internal compartment; an artificial adeno-associated virus (AAV) vector located within the internal compartment of the liposome, wherein the AAV vector includes DNA encoding a biologically active agent; one or more blood-brain barrier and brain cell membrane targeting agents; and one or more conjugation agents wherein each targeting agent is connected to the exterior surface of the liposome via at least one of the conjugation agents.

In another aspect, the present invention provides a composition for delivering single-stranded DNA across a blood-brain barrier for expression in the brain, the composition comprising: a) a receptor-specific liposome, wherein the receptor-specific liposome comprises: a liposome having an exterior surface and an internal compartment; an artificial adeno-associated virus (AAV) vector located within the internal compartment of the liposome, wherein the artificial AAV vector comprises single stranded DNA encoding a biologically active agent; one or more blood-brain barrier and brain cell membrane targeting agents; and one or more conjugation agents wherein each targeting agent is connected to the exterior surface of the liposome via at least one of the conjugation agents; or b) a receptor-specific nanocontainer, wherein the receptor-specific nanocontainer comprises: a nanocontainer having an exterior surface and an internal compartment; an artificial adeno-associated virus (AAV) vector located within the internal compartment of the nanocontainer, wherein the artificial AAV vector comprises single stranded DNA encoding a biologically active agent; one or more receptor specific targeting agents that target the receptor located on the cell; and one or more conjugation agents wherein each targeting agent is connected to the exterior surface of the nanocontainer via at least one of the conjugating agents; or c) an artificial adeno-associated virus (AAV) vector comprising single stranded DNA encoding a biologicially active agent; and a component to deliver at least the single-stranded DNA across the blood brain barrier, wherein in a), b) or c): said single stranded DNA encoding the biologically active agent is SEQ ID NO: 2; or said AAV is selected from SEQ ID NOs: 8-11. Compositions of the invention for use in the treatment of Alzheimer's disease form yet further aspects of the invention.

Also described herein is a composition that includes a receptor-specific liposome, wherein the receptor-specific liposome includes: a liposome having an exterior surface and an internal compartment; an artificial adeno-associated virus (AAV) vector located within the internal compartment of the liposome, wherein the AAV vector includes DNA encoding a biologically active agent; one or more blood-brain barrier and brain cell membrane targeting agents; and one or more conjugation agents wherein each targeting agent is connected to the exterior surface of the liposome via at least one of the conjugation agents.

Also described herein is a composition for delivering DNA to a cell. The composition includes a receptor-specific nanocontainer, wherein the receptor-specific nanocontainer includes: a nanocontainer having an exterior surface and an internal compartment; an artificial adeno-associated virus (AAV) vector located within the internal compartment of the nanocontainer, wherein the AAV vector includes DNA encoding a biologically active agent; one or more receptor specific targeting agents that target the receptor located on the cell; and one or more conjugation agents wherein each targeting agent is connected to the exterior surface of the nanocontainer via at least one of the conjugation agents.

Also described herein is a composition for delivering DNA across a blood-brain barrier for expression in the brain. The composition includes: an artificial adeno-associated virus (AAV) vector including DNA encoding a biologically active agent; and a component to deliver at least the DNA across the blood-brain barrier.

In another aspect, the present invention provide artificial AAV vectors for delivering DNA encoding a biologically active agent, and methods of making and using such vectors. Thus, the present invention provides an artificial adeno-associated virus (AAV) vector comprising, in 5-prime to 3-prime order: a 5-prime AAV-ITR; a single stranded DNA encoding a biologically active agent; an internal AAV-ITR; a reverse complement of the DNA encoding the biologically active agent; and a 3-prime AAV-ITR, wherein: a) said single stranded DNA encoding the biologically active agent is encoded by SEQ ID NO: 2; or b) said artificial AAV is selected from SEQ ID NOs: 8-11.

Also described herein is an artificial AAV vector including, in 5-prime to 3-prime order: a 5-prime AAV-ITR; a single stranded DNA encoding a biologically active agent; an internal AAV-ITR; a reverse complement of the single stranded DNA encoding the biologically active agent: and a 3-prime AAV-ITR. Methods of making such vectors are also provided.

Also described herein is an artificial adeno-associated virus (AAV) vector for delivery of a linear, double stranded DNA encoding a biologically active agent, the artificial AAV vector including the linear, double stranded DNA having AAV-ITRs at the 5-prime and 3-prime ends of each strand. Preferably, the artificial AAV vector has been thermally treated in at least one heating and cooling cycle.

The present invention can offer advantages over other methods of delivering biologically active agents including, for example, conventional enhanced delivery, stereotactic neurosurgical delivery of viral or non-viral vectors, and/or intravenous delivery of a composition for carrying plasmid DNA or RNA across the blood brain barrier.

The use of an artificial AAV vector to deliver a gene or a gene-suppressing agent to a patient's brain can have many advantages over the delivery of plasmid DNA, or the delivery of actual AAV virus particles. One possible advantage of delivering the DNA of an AAV vector to the brain, rather than a plasmid DNA, is that expression of AAV-delivered gene constructs in the primate brain is known to persist for at least 3 to 4 years, whereas expression of gene constructs from plasmids is temporary. The advantages of delivering the DNA of a synthetic AAV vector over delivery of AAV virus particles can be several. First, delivery of just the DNA can circumvent the delivery of AAV viral capsids to the patient's brain. Since it is the AAV viral capsid proteins that are most likely to trigger an immune response, dispensing with the need to deliver viral particles can avoid most of the risk of adverse immune reactions to the therapy. Further, delivery of the DNA can circumvent the need to produce complete AAV particles, a difficult manufacturing step that requires the use of specially engineered and cultured cells to make the AAV capsids and package the DNA into the virus capsids. Finally, delivery of DNA rather than AAV particles can circumvent the natural limitation on the length of the DNA that can be packaged inside AAV capsids, which is about 4,700 bases of DNA. Although this size limitation is not a problem for delivery of constructs for gene suppression (e.g., DNA coding for small, interfering RNA), it can be a limitation for delivery of missing genes, if the sequence for the missing gene is longer than 4,700 bases, which has been noted as a limitation on the use of AAV as a vector for gene therapy.

### Definitions

By "alpha-synuclein, BACE1 (including variants thereof, e.g. variants A, B, C, and D), huntingtin, ataxin-1, ataxin-3, and/or atrophin-1 proteins" is meant, a protein or a mutant protein derivative thereof, including the amino-acid sequence expressed and/or encoded by alpha-synuclein (Parkinson's disease), beta-site APP-cleaving enzyme (BACE1 (including variants thereof, e.g. variants A, B, C, and D)) (Alzheimer's disease), huntingtin (Huntington's disease), ataxin-1 (Spinocerebellar Ataxia Type 1), ataxin-3 (Spinocerebellar Ataxia Type 3 or Machado-Joseph's Disease), and/or dentatorubral pallidoluysian atrophy (DRPLA) genes, respectively.

As used herein "cell" is used in its usual biological sense, and does not refer to an entire multicellular organism. The cell may be present in an organism which may be a human but is preferably of mammalian origin, e.g., such as humans, cows, sheep, apes, monkeys, swine, dogs, cats, and the like. However, several steps of producing small interfering RNA may require use of prokaryotic cells (e.g., bacterial cell) or eukaryotic cell (e.g., mammalian cell) and thereby are also included within the term "cell".

By "complementarity" it is meant that a molecule including one or more nucleic acids (DNA or RNA) can form hydrogen bond(s) with another molecule including one or more nucleic acids by either traditional Watson-Crick pairing or other non-traditional types.

The term "equivalent" DNA is meant to include naturally occurring DNA having homology (partial or complete) to DNA encoding for the same protein in a different organism (e.g., human, rodent, primate, rabbit, pig, and microorganisms). The equivalent DNA sequence can also include regions such as the 5'-untranslated region, the 3'- untranslated region, introns, intron-exon junctions, small interfering RNA targeted site and the like, optionally incorporated into the DNA of infective viruses, such as adeno-associated virus (AAV).

The term "functional equivalent" refers to any derivative that is functionally similar to the reference sequence or protein. In particular, the term "functional equivalent" includes derivatives in which the nucleotide bases(s) have been added, deleted, or replaced without a significant adverse effect on biological function.

As used herein, the term "biologically active" as used with "agent" or "siRNA" means that the agent or siRNA can modify a cell in any way including, for example, modifying the metabolism of the cell, the structure of the cell, the function of the cell, and/or permit the cell containing the agent or siRNA to be detected. Examples of biologically active agents and/or siRNAs include, for example, polynucleotides, polypeptides, and combinations thereof. A biologically active agent or siRNA may be therapeutic (i.e., able to treat or prevent a disease) or non-therapeutic (i.e., not directed to the treatment or prevention of a disease). Non-therapeutic biologically active compounds include detection or diagnostic agents including, for example, markers that can be used for detecting the presence of a particular cell, distinguishing cells, and/or detecting whether a targeting group is functioning to target a particular tissue. As used herein, the term "polynucleotide" or "nucleic acid molecule" refers to a polymeric form of nucleotides of any length, either ribonucleotides or deoxynucleotides, and includes both double- and single-stranded DNA and RNA, and combinations thereof. A polynucleotide may include nucleotide sequences having different functions including, for example, coding sequences and non-coding sequences such as regulatory sequences. Coding sequence, non-coding sequence, and regulatory sequence are defined below. A polynucleotide can be obtained directly from a natural source, or can be prepared with the aid of recombinant, enzymatic, or chemical techniques. A polynucleotide can be linear or circular in topology. A polynucleotide can be, for example, a portion of a vector, or a fragment.

A "coding sequence" or a "coding region" is a polynucleotide that encodes a polypeptide and, when placed under the control of appropriate regulatory sequences, expresses the encoded polypeptide. The boundaries of a coding region are generally determined by a translational start codon at its 5-prime end and a translational stop codon at its 3-prime end. A regulatory sequence is a nucleotide sequence that regulates expression of a coding region to which it is operably linked. Nonlimiting examples of regulatory sequences include promoters, transcriptional initiation sites, translational start sites, translational stop sites, transcriptional terminators (including, for example, polyadenylation signals), and intervening sequences (introns). "Operably linked" refers to a juxtaposition wherein the components so described are in a relationship permitting them to function in their intended manner. A regulatory sequence is "operably linked" to a coding region when it is joined in such a way that expression of the coding region is achieved under conditions compatible with the regulatory sequence. The term "gene" is meant to include a polynucleotide that includes a coding sequence or coding region.

The term "vector" is commonly known in the art and defines a plasmid DNA, phage DNA, viral DNA and the like, which can serve as a DNA vehicle into which DNA of the present invention can be inserted, and from which RNA can be transcribed. The term "vectors" refers to any of these nucleic acid and/or viral-based techniques used to deliver a desired nucleic acid. Numerous types of vectors exist and are well known in the art.

The term "expression" defines the process by which a gene is transcribed into RNA (transcription); the RNA may be further processed into a mature small interfering RNA, or into mRNA from which a cell can produce a protein.

The terminology "expression vector" defines a vector or vehicle as described above but designed to enable the expression of an inserted sequence following transformation into a host. The cloned gene (inserted sequence) is usually placed under the control of control element sequences such as promoter sequences. The placing of a cloned gene under such control sequences is often referred to as being operably linked to control elements or sequences.

"Promoter" refers to a DNA regulatory region capable of binding directly or indirectly to RNA polymerase in a cell and initiating transcription of a downstream (3-prime direction) coding sequence. For purposes of the present invention, the promoter is bound at its 3-prime terminus by the transcription initiation site and extends upstream (5-prime direction) to include the minimum number of bases or elements necessary to initiate transcription at levels detectable above background. Within the promoter will be found a transcription initiation site (conveniently defined by mapping with S1 nuclease), as well as protein binding domains (consensus sequences) responsible for the binding of RNA polymerase. Eukaryotic promoters will often, but hot always, contain "TATA" boxes and "CAAT" boxes. Prokaryotic promoters contain -10 and -35 consensus sequences, which serve to initiate transcription.

By "homology" it is meant that the nucleotide sequence of two or more nucleic acid molecules is partially or completely identical.

By "highly conserved sequence region" it is meant that a nucleotide sequence of one or more regions in a target gene does not vary significantly from one generation to the other or from one biological system to the other.

By the term "inhibit" or "inhibitory" it is meant that the activity of the target genes or level of mRNAs or equivalent RNAs encoding target genes is reduced below that observed in the absence of the provided small interfering RNA. Preferably the inhibition is at least 10% less, 25% less, 50% less, 75% less, 85% less, or 95% less than in the absence of the small interfering RNA.

By "inhibited expression" or "protein suppression" it is meant that the reduction of alpha-synuclein, BACE1 (including variants thereof, e.g. variants A, B, C, and D), huntingtin, ataxin-1, ataxin-3 and/or atrophin-1 mRNA levels and thus reduction in the level of the respective protein to relieve, to some extent, the symptoms of the disease or condition.

By "RNA" is meant ribonucleic acid, a molecule consisting of ribonucleotides connected via a phosphate-ribose (sugar) backbone. By "ribonucleotide" is meant guanine, cytosine, uracil, or adenine or some a nucleotide with a hydroxyl group at the 2' position of a beta-D- ribo-furanose moiety. As is well known in the art, the genetic code uses thymidine as a base in DNA sequences and uracil in RNA. One skilled in the art knows how to replace thymidine with uracil in a written nucleic acid sequence to convert a written DNA sequence into a written RNA sequence, or vice versa.

By "patient" is meant an organism, which is a donor or recipient of explanted cells or the cells themselves. "Patient" also refers to an organism to which the nucleic acid molecules of the invention can be administered. Preferably, a patient is a mammal or mammalian cells, e.g., such as humans, cows, sheep, apes, monkeys, swine, dogs, cats, and the like, or cells of these animals used for transplantation. More preferably, a patient is a human or human cells.

The term "synuclein" refers to an alpha-synuclein (especially human or mouse) or beta-synuclein (especially human or mouse). An example of a full nucleotide sequence encoding human alpha-synuclein is available under Genbank Accession No. AF163864. Examples of variants of the human alpha-synuclein sequence are available under Genbank Accession Nos. NM_000345 and NM_007308. An example of mouse alpha-synuclein is available under Genbank Accession No. AF163865.

The term "BACE1" refers to a beta-site amyloid precursor protein cleaving enzyme type 1 (especially human or mouse). Several variants of BACE1 have been sequenced, including variants A, B, C, and D. In some scientific literature, BACE1 is also known as ASP2 and Memapsin2. Examples of full nucleotide sequences encoding human BACE1, and variants related thereto, are available under Genbank Accession Nos. NM_138971, NM_138972, NM_138973, and NM_012104. An example of a mouse homolog is available under Genbank Accession No. NM_011792.

The term "huntingtin" refers to a protein product encoded by the Huntington's Disease gene (IT-15) (especially human or mouse). An example of a full nucleotide sequence encoding human IT-15 is available under Genbank Accession No. AH003045. An example of a mouse sequence is available under Genbank Accession No. U24233.

The term "ataxin-1" refers to a protein product encoded by the Spinocerebellar Ataxia Type 1 gene (especially human or mouse). An example of a full nucleotide sequence encoding human SCA1 is available under Genbank Accession No. NM_000332. An example of a mouse SCA1 is available under Genbank Accession No. NM_009124.

The term "ataxin-3" refers to a protein product encoded by the Spinocerebellar Ataxia Type 3 gene (especially human or mouse). Examples of full nucleotide sequences encoding human SCA3 are available under Genbank Accession Nos. NM_004993 (splice variant 1) and NM_030660 (splice variant 2). An example of a sequence for a mouse homolog is not yet available.

The term "atrophin-1" refers to a protein product encoded by the dentatorubral pallidoluysian atrophy (DRPLA) gene (especially human or mouse). An example of a full nucleotide sequence encoding human DRPLA is available under Genbank Accession No. XM_032588. An example of a mouse sequence is available under Genbank Accession No. XM_132846.

The term "inborn error of metabolism" refers to rare genetic disorders in which the body cannot turn food into energy (i.e., metabolize food) normally. The disorders are usually caused by defects in the enzymes involved in the biochemical pathways that break down food components.

The term "lysosomal storage disorder" (LSD) refers to an inherited disease characterized by a defect in the functional expression of any of the lysosomal enzymes.

The term "lysosome" refers to a eukaryotic membrane-bound organelle containing numerous different digestive enzymes which can break down many substances.

The term "modification" includes derivatives substantially similar to the reference sequence or protein. '

By "small interfering RNA" is meant a nucleic acid molecule which has complementarity in a substrate binding region to a specified gene target, and which acts to specifically guide enzymes in the host cell to cleave the target RNA. That is, the small interfering RNA by virtue of the specificity of its sequence and its homology to the RNA target is able to cause cleavage of the RNA strand and thereby inactivate a target RNA molecule because it is no longer able to be translated into protein. These complementary regions allow sufficient hybridization of the small interfering RNA to the target RNA and thus permit cleavage. One hundred percent complementarity is often necessary for biological activity and therefore is preferred, but complementarity as low as 90% may also be useful in this invention. The specific small interfering RNA described in the present application are not meant to be limiting and those skilled in the art will recognize that all that is important in a small interfering RNA of this invention is that it have a specific substrate binding site which is complementary to one or more of the target nucleic acid regions.

Small interfering RNAs are double stranded RNA agents that have complementarity to (i.e., able to base-pair with) a portion of the target RNA (generally messenger RNA). Generally, such complementarity is 100%, but can be less if desired, such as 90%, 91%, 92%, 93%, 94%, 95%, 96%, 97%, 98%, or 99%. For example, 19 bases out of 21 bases may be base-paired. In some instances, where selection between various allelic variants is desired, 100% complementarity to the target gene is required in order to effectively discern the target sequence from the other allelic sequence. When selecting between allelic targets, choice of length is also an important factor because it is the other factor involved in the percent complementarity and the ability to differentiate between allelic differences.

The small interfering RNA sequence needs to be of sufficient length to bring the small interfering RNA and target RNA together through complementary base-pairing interactions. The small interfering RNA of the invention may be of varying lengths. The length of the small interfering RNA is preferably greater than or equal to 10 nucleotides and of sufficient length to stably interact with the target RNA; specifically 15-30 nucleotides; more specifically any integer between 15 and 30 nucleotides, such as 15, 16, 17, 18, 19, 20, 21, 22, 23, 24, 25, 26, 27, 28, 29, and 30. By "sufficient length" is meant an oligonucleotide of greater than or equal to 15 nucleotides that is of a length great enough to provide the intended function under the expected condition. By "stably interact" is meant an interaction of a small interfering RNA with a target nucleic acid (e.g., by forming hydrogen bonds with complementary nucleotides in the target under physiological conditions).

A "reverse complement" of a DNA strand in a 5-prime to 3-prime direction is a DNA strand in the reverse order with the corresponding complementary bases according to Watson-Crick or other base pairing rules.

### BRIEF DESCRIPTION OF THE DRAWINGS

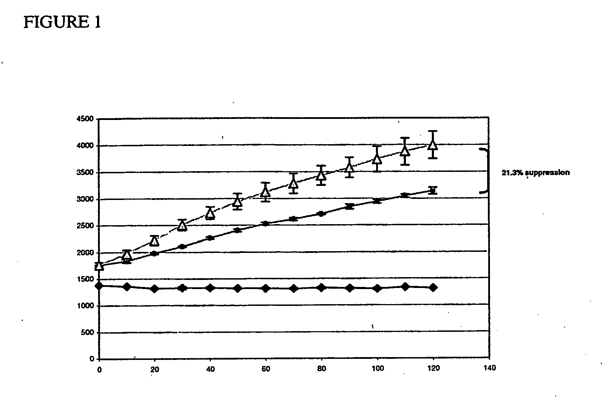

Figure 1 illustrates total BACE enzyme activity in protein extracts from transfected HEK293 cells through a graphical representation of measured fluorescence (y-axis, fluorescence units) versus time (x-axis, minutes) as described in Example 4. The values for background (assay reagents only, no cells) are represented by the symbol "◆." The values for MB1749 siRNA and pTracerBace are represented by the symbol "●." The values for pSilencer control and pTracerBace are represented by the symbol "Δ."

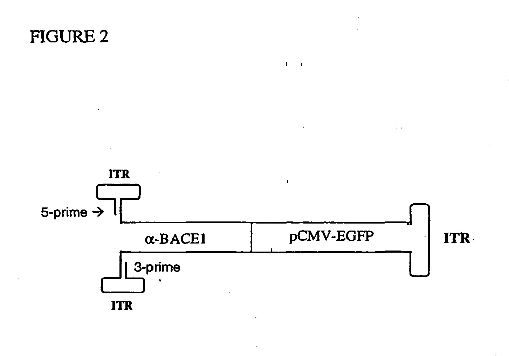

Figure 2 is a schematic representation of one embodiment of a self-complementary artificial AAV vector for delivery of a single stranded DNA. The artificial AAV vector includes, in 5-prime to 3-prime order: a 5-prime AAV-ITR (ITR); a single stranded DNA (α-BACE1/pCMV-EGFP); an internal AAV-ITR (ITR); a reverse complement of the single stranded DNA (α-BACE1/pCMV-EGFP); and a 3-prime AAV-TTR (ITR).

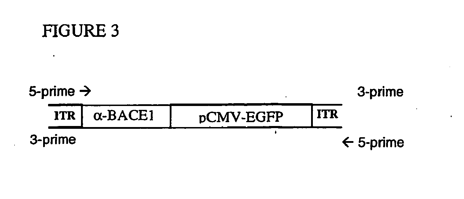

Figure 3 is a schematic representation of one embodiment of an artificial AAV vector for delivery of a linear, double stranded DNA. The linear, double stranded DNA (α-BACE1/pCMV-EGFP) has AAV-TTRs (ITR) at the 5-prime and 3-prime ends of each strand.

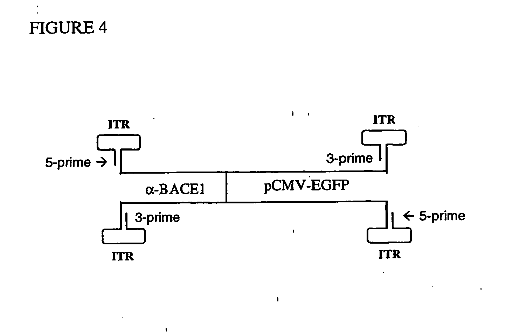

Figure 4 is a schematic representation of one embodiment of an artificial AAV vector for delivery of a linear, double stranded DNA as illustrated in Figure 3 that has been thermally treated in at least one heating and cooling cycle. The schematic representation illustrates a secondary structure of the ITRs in which the ITRs have folded so as to allow the self-complementary portions of each ITR to internally hybridize. '

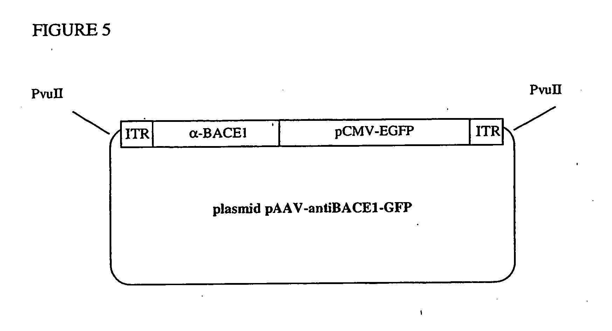

Figure 5 is a schematic representation of one embodiment of a plasmid, pAAV-antiBACE1-GFP, as produced following steps 1 through step 5 of Example 5, and as used in Example 7. The plasmid includes between two PvuII restriction sites, in 5-prime to 3-prime order, a 5-prime AAV-ITR (ITR), a DNA segment (α-BACE1/pCMV-EGFP), and a 3-prime AAV-TTR (ITR).

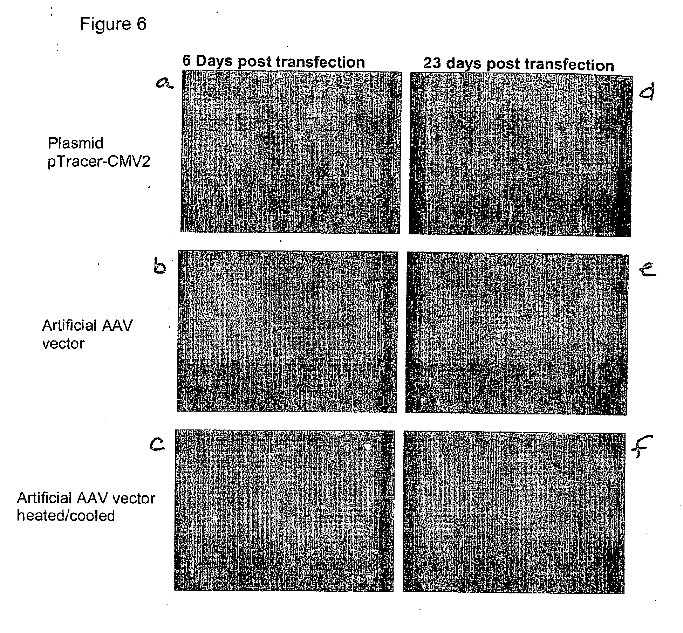

Figure 6 illustrates photographs of fluorescent microscopy images of HEK293T cells that have been transfected with pTRACER CMV2 circular plasmid (Figures 6a and 6d); a linear, double-stranded artificial AAV vector of the subject invention (Figures 6b and 6e); or a linear, double-stranded artificial AAV vector of the subject invention that had been heated to 65 degrees Centigrade and cooled to room temperature (Figures 6c and 6f). The photographs show the expression of EGFP in the HEK293T cells 6 days post-transfection (Figures 6a-6c) and 23 days post-transfection (Figures 6d-6f) as described in Example 7.

### DETAILED DESCRIPTION OF CERTAIN EMBODIMENTS

The present invention provides medical systems and methods for delivering DNA to a target site (e.g., to a cell or across the blood-brain barrier). The cell may be *in vivo* or *ex vivo.* As used herein, the term *"ex vivo"* refers to a cell that has been removed, for example, isolated, from the body of a subject. *Ex vivo* cells include, for example, primary cells (e.g., cells that have recently been removed from a subject and are capable of limited growth or maintenance in tissue culture medium), and cultured cells (e.g., cells that are capable of extended growth or maintenance in tissue culture medium). As used herein, the term *"in vivo"* refers to a cell that is within the body of a subject.

The medical systems include a neurovascular catheter having its distal end positioned in a blood vessel supplying a patient's brain. Optionally, the system further includes an implantable pump for delivery of the composition to the patient's blood stream. The medical system further includes a means for delivering to the catheter a composition as described herein. Methods of delivering such compositions to a cell or across the blood-brain barrier for expression in the brain are also described herein.

In brief, compositions disclosed and used in the present invention include an artificial single-stranded adeno-associated virus (AAV) vector, including DNA encoding a biologically active agent; and a component (e.g., a receptor-specific liposome as described herein) that delivers at least the DNA across the blood-brain barrier. In some embodiments, the artificial AAV vector includes, in 5-prime to 3-prime order: a 5-prime AAV inverted terminal repeat (AAV-ITR); a single stranded DNA encoding the biologically active agent; and a 3-prime AAV-ITR. In other embodiments, the artificial AAV vector includes, in 5-prime to 3-prime order: a 5-prime AAV-ITR; a single stranded DNA encoding a biologically active agent; an internal AAV-ITR; a reverse complement of the single stranded DNA encoding the biologically active agent: and a 3-prime AAV-ITR. In other examples described herein, the artificial AAV vector includes a linear, double stranded DNA having AAV-ITRs at the 5-prime and 3-prime ends of each strand. Preferably, the artificial AAV vector does not include a coding sequence to encode a capsid, and thus, the preferred vectors are not encapsulated in a viral capsid structure. Methods of making artificial AAV vectors are also disclosed.

For embodiments in which the DNA encodes a small interfering RNA, the compositions can be useful for treating, among other things, various neurodegenerative disorders caused by a pathogenic protein. For embodiments in which the DNA encodes a protein, the compositions can be useful for treating, among other things, various neurological diseases caused by the absence of the protein.

In some embodiments, the compositions include a receptor-specific liposome and a pharmaceutically acceptable carrier for the receptor-specific liposome, wherein the receptor-specific liposome includes: a liposome having an exterior surface and an internal compartment; the artificial adeno-associated virus (AAV) vector located within the internal compartment of the liposome; one or more blood-brain barrier and brain cell membrane targeting agents; and one or more conjugation agents, wherein each targeting agent is connected to the exterior surface of the liposome via at least one of the conjugation agents.

In other embodiments, the compositions include a receptor-specific nanocontainer (i.e., a container having at least one dimension on the order of a few nanometers or less) and a pharmaceutically acceptable carrier for the receptor-specific nanocontainer, wherein the receptor-specific nanocontainer includes: a nanocontainer having an exterior surface and an internal compartment; an artificial adeno-associated virus (AAV) vector located within the internal compartment of the nanocontainer; one or more receptor specific targeting agents that target the receptor located on the cell; and one or more conjugation agents, wherein each targeting agent is connected to the exterior surface of the nanocontainer via at least one of the conjugation agents.

### MEDICAL DEVICES

The present invention provides medical devices that include a neurovascular catheter and an optional implantable pump for delivery of the composition into a patient's blood stream. The distal, delivery end of the neurovascular catheter is positioned in a blood vessel supplying the brain. For acute use, the proximal end of the neurovascular catheter would remain outside the patient's body at the point of introduction (e.g., the femoral artery) and used by the physician to deliver the composition in a suitable fluid solution to the patient's brain. Although the delivery in this case is acute, the therapy may nevertheless be long-lasting as described herein below.

Alternatively, the proximal end of the neurovascular catheter can be attached to the optional implantable pump, and both the pump and catheter chronically implanted in the body. In the latter case, the pump provides a "catheter access port" through which the physician can transcutaneously make repeated bolus injections of the composition through the catheter into the blood vessel supplying the patient's brain. The pump provides a fluid reservoir used to supply heparinized saline, dilute tissue plasminogen activator (tPA), or a similar agent that is continuously pumped at a low rate through the neurovascular catheter in between uses of the catheter for bolus injections. The purpose is to prevent blood clots from forming at the distal end of the catheter, occluding the catheter lumen and posing a risk of embolic stroke to the patient.

### NEURODEGENERATIVE DISORDERS CAUSED BY A PATHOGENIC PROTEIN

For several neurodegenerative diseases, such as Parkinson's disease, Alzheimer's disease, Huntington's disease, Spinocerebellar Ataxia Type 1 and Type 3, and dentatorubral pallidoluysian atrophy (DRLPA), proteins involved in the overall pathogenic progression of the disease have been identified. There is currently no cure for these neurodegenerative diseases. These diseases are progressively debilitating and most are ultimately fatal.

Further problematic of these neurodegenerative diseases (especially Alzheimer's disease and Parkinson's disease) is that their prevalence continues to increase, thus creating a serious public health problem. Recent studies have pointed to alpha-synuclein (Parkinson's disease), beta- amyloid-cleaving enzyme 1 (BACE1 (including variants thereof, e.g. variants A, B, C, and D)) (Alzheimer's disease), huntingtin (Huntington's disease), and ataxin 1 (Spinocerebellar Ataxia Type 1) as major factors in the pathogenesis of each of these diseases, respectively.

The neurodegenerative process in Parkinson's disease and Alzheimer's disease is characterized by extensive loss of selected neuronal cell populations accompanied by synaptic injury and astrogliosis. Pathological hallmarks of Alzheimer's disease include formation of amyloid plaques, neurofibrillary tangles and neuropil thread formation; pathological hallmarks of Parkinson's diseases include the formation of intraneuronal inclusions called Lewy bodies and the loss of dopaminergic neurons in the substantia nigra. Although the mechanisms triggering cell dysfunction and death are unclear, the prevailing view is that neurodegeneration results from toxic effects subsequent to the accumulation of specific neuronal cell proteins, such as alpha-synuclein (Parkinson's disease) and amyloid precursor protein (APP) (Alzheimer's disease - processed into beta-amyloid by BACE1 (including variants thereof, e.g. variants A, B, C, and D)).

Alpha-synuclein has been implicated in Parkinson's disease because it is abundantly found in Lewy Bodies, its overexpression in transgenic mice leads to Parkinson's disease-like pathology, and mutations within this molecule are associated with familial Parkinson's disease. Alpha-synuclein, which belongs to a larger family of molecules including beta and gamma-synuclein, is a 140 amino acid non-amyloid synaptic protein which is a precursor of the 35 amino acid non-amyloid component protein found in amyloid plaques.

Alzheimer's disease is a progressive degenerative disorder of the brain characterized by mental deterioration, memory loss, confusion, and disorientation. Among the cellular mechanisms contributing to this pathology are two types of fibrillar protein deposits in the brain: intracellular neurofibrillary tangles composed of polymerized tau protein, and abundant extracellular fibrils including largely beta-amyloid. Beta-amyloid, also known as Abeta, arises from the proteolytic processing of the amyloid precursor protein (APP) at the beta- and gamma- secretase cleavage sites giving rise to the cellular toxicity and amyloid-forming capacity of the two major forms of Abeta (Abeta₄₀ and Abeta₄₂). Thus, preventing APP processing into plaque-producing forms of amyloid may critically influence the formation and progression of the disease, making BACE1 (including variants thereof, e.g. variants A, B, C, and D) a clinical target for inhibiting or arresting this disease. Similar reports suggest presenilins are candidate targets for redirecting aberrant processing.

Huntington's disease is a fatal, hereditary neurodegenerative disorder characterized by involuntary "ballistic" movements, depression, and dementia. The cause has been established to be a mutation in a single gene consisting of an excessively long series of CAG trinucleotide sequences in the DNA. This CAG repeat is in the coding region of the gene. Thus, the resulting huntingtin protein also contains an excessively long region made of the amino acid glutamine, for which "CAG" encodes. Shortly after this mutation was pinpointed as the cause of Huntington's disease, similar CAG repeat expansions in other genes were sought and found to be the cause of numerous other fatal, hereditary neurodegenerative diseases. The list of these so-called "polyglutamine" diseases now includes at least eleven more, including: spinocerebellar ataxia type 1, type 2, and type 3, spinobulbar muscular atrophy (SBMA or Kennedy's disease) and dentatorubral pallidoluysian atrophy (DRPLA). Although the particular gene containing the expanded CAG repeat is different in each disease, it is the production of an expanded polyglutamine protein in the brain that causes each one. Symptoms typically emerge in early to middle-aged adulthood, with death ensuing 10 to 15 years later. No effective treatments for these fatal diseases currently exist.

There is considerable evidence suggesting that shutting off production of the abnormal protein in neurons will be therapeutic in polyglutamine diseases. The cause of these diseases is known to be the gain of a new function by the mutant protein, not the loss of the protein's original function. Mice harboring the human, expanded transgene for spinocerebellar ataxia type 1 (SCA1) become severely ataxic in young adulthood (Clark et al., Journal of Neuroscience 17:7385-7395 (1997)), but mice in which the corresponding mouse gene has been knocked out do not suffer ataxia or display other major abnormalities (Matilla et al., Journal of Neuroscience 18:5508-5516 (1998)). Transgenic mice for SCA1 in which the abnormal ataxin1 protein is produced but has been genetically engineered to be incapable of entering the cell's nucleus do not develop ataxia (Klement et al., Cell 95:41-53 (1998)). Finally, a transgenic mouse model of Huntington's disease has been made in which the mutant human transgene has been engineered in a way that it can be artificially "turned off" by administering tetracycline (Normally, in mice and humans, administration of this antibiotic would have no effect on the disease). After these mice have begun to develop symptoms, shutting off production of the abnormal protein production by chronic administration of tetracycline leads to an improvement in their behavior (Yamamoto et al., Cell 101:57-66 (2000)). This suggests that reducing expression of the abnormal huntingtin protein in humans might not only prevent Huntington's disease from progressing in newly diagnosed patients, but may improve the quality of life of patients already suffering from its symptoms.

Various groups have been recently studying the effectiveness of siRNAs. Caplen et al., in Human Molecular Genetics, 11(2):175-184 (2002), assessed a variety of different double stranded RNAs for their ability to inbibit cell expression of mRNA transcripts of the human androgen receptor gene containing different CAG repeats. Their work found gene-specific inhibition occurred with double stranded RNAs containing CAG repeats only when flanking sequences to the CAG repeats were present in the double stranded RNAs. They were also able to show that constructed double stranded RNAs were able to rescue caspase-3 activation induced by expression of a protein with an expanded polyglutamine region. Xia et al., in Nature Biotechnology, 20:1006-1010 (2002), demonstrated the inhibition of polyglutamine (CAG) expression of engineered neural PC12 clonal cell lines that express a fused polyglutamine-fluorescent protein using constructed recombinant adenovirus expressing siRNAs targeting the mRNA encoding green fluorescent protein.

The design and use of small interfering RNA complementary to mRNA targets that produce particular proteins is a recent tool employed by molecular biologists to prevent translation of specific mRNAs. Other tools used by molecular biologists to interfere with protein expression prior to translation involve cleavage of the mRNA sequences using ribozymes against therapeutic targets for Alzheimer's disease (*see, for example,* PCT International Application Publication No. WO 01/16312 A2 (McSwiggen et al.)) and Parkinson's disease (*see, for example,* PCT International Application Publication Nos. WO 99/50300 A1 (Trojanowski et al.) and WO 01/60794 A2 (Eliezer)). PCT International Application Publication No. WO 2004/047872 A2 (Kaemmerer) and U.S. Patent Application Publication No. 2004/0220132 A1 (Kaemmerer) disclose devices, small interfering RNA, and methods for treating a neurodegenerative disorder including the steps of surgically implanting a catheter so that a discharge portion of the catheter lies adjacent to a predetermined infusion site in a brain, and discharging through the discharge portion of the catheter a predetermined dosage of at least one substance that inhibits production of at least one neurodegenerative protein. PCT International Application Publication No. WO 2004/047872 A2 (Kaemmerer) and U.S. Patent Application Publication No. 2004/0220132 A1 (Kaemmerer) further disclose small interfering RNA vectors, and methods for treating neurodegenerative disorders such as Alzheimer's disease, Parkinson's disease, Huntington's disease, Spinocerebellar Ataxia Type 1, Type 2, Type 3, and/or dentatorubral-pallidoluysian atrophy.

### SMALL INTERFERING RNA (siRNA)

As previously indicated, the small interfering RNA (or siRNA) described herein, is a segment of double stranded RNA that is from 15 to 30 nucleotides in length. It is used to trigger a cellular reaction known as RNA interference. In RNA interference, double-stranded RNA is digested by an intracellular enzyme known as Dicer, producing siRNA duplexes. The siRNA duplexes bind to another intracellular enzyme complex which is thereby activated to target whatever mRNA molecules are homologous (or complementary) to the siRNA sequence. The activated enzyme complex cleaves the targeted mRNA, destroying it and preventing it from being used to direct the synthesis of its corresponding protein product. Recent evidence suggests that RNA interference is an ancient, innate mechanism for not only defense against viral infection (many viruses introduce foreign RNA into cells) but also gene regulation at very fundamental levels. RNA interference has been found to occur in plants, insects, lower animals, and mammals, and has been found to be dramatically more effective than other gene silencing technologies, such as antisense or ribozymes. Used as a biotechnology technique, siRNA involves introducing into cells (or causing cells to produce) short, double-stranded molecules of RNA similar to those that would be produced by the Dicer enzyme from an invading double-stranded RNA virus. The artificially-triggered RNA interference process then continues from that point.

To deliver a small interfering RNA to a patient's brain, a preferred method will be to introduce the DNA encoding for the siRNA, rather than the siRNA molecules themselves, into the cells of the brain. The DNA sequence encoding for the particular biologically active siRNA can be specified upon knowing (a) the sequence for a small portion of the target mRNA (available in public human genome databases; *see, also,* Chi et al., Proc. Nat. Acad. Sci. USA, 100:6343-6346 (2003) and the world wide web at rockefeller.edu/labheads/tuschl/sima.html and at dharmacon dot com), and (b) well-known codon usage to specify DNA that will result in production of a corresponding RNA sequence when the DNA is transcribed by cells.

The DNA sequence, once specified, can be constructed in the laboratory from synthetic molecules ordered from a laboratory Supplier, and inserted using standard molecular biology methods into one of several alternative "vectors" for delivery of DNA to cells. Once delivered into the neurons of the patient's brain, those neurons will themselves produce the RNA that becomes the biologically active siRNA, by transcribing the inserted DNA into RNA. The result will be that the cells themselves produce the siRNA that will silence the targeted gene. The result will be a reduction of the amount of the targeted protein produced by the cell.

In accordance with the present invention, small interfering RNA against specific mRNAs produced in the affected cells prevent the production of the disease related proteins in neurons. In accordance with the present invention is the use of specifically tailored vectors designed to deliver small interfering RNA to targeted cells. The success of the designed small interfering RNA is predicated on their successful delivery to the targeted cells of the brain to treat the neurodegenerative diseases.

Small interfering RNA have been shown to be target specific mRNA molecules in human cells. Small interfering RNA vectors can be constructed to transfect human cells and produce small interfering RNA that cause the cleavage of the target RNA and thereby interrupt production of the encoded protein.

A small interfering RNA vector of the present invention will prevent production of the pathogenic protein by suppressing production of the neuropathogenic protein itself or by suppressing production of a protein involved in the production or processing of the neuropathogenic protein. Repeated administration of the biologically active agent to the patient may be required to accomplish the change in a large enough number of neurons to improve the patient's quality of life. Within an individual neuron, however, the change is longstanding enough to provide a therapeutic benefit. The desperate situation of many patients suffering from neurodegenerative disorders, such as Alzheimer's disease, Parkinson's disease, Huntington's disease, or Spinocerebellar Ataxia Type 1 provides a strong likelihood that the benefit from the therapy will outweigh the risks of the therapy delivery and administration. While it may be possible to accomplish some reduction in the production of neuropathogenic proteins with other biologically active agents and routes of administration, development of successful therapies involving direct *in vivo* transfection of neurons may provide the best approach based on delivery of small interfering RNA vectors to targeted cells.

Exemplary DNA sequences encoding for siRNA are listed in Table 1.

**TABLE 1: Sequences of biologically active siRNA:**

| *Disease* | *Target* | *DNA sequence encoding for siRNA* |
|---|---|---|
| Alzheimer's disease | BACE1 | 5' - AAGGGTGTGTATGTGCCCTAC - 3' (SEQ ID NO:1) |
| Alzheimer's disease | BACE1 | 5' - AAGACTGTGGCTACAACATTC - 3' (SEQ ID NO:2) |
| Huntington's disease | Huntingtin | 5' - AAGGTTACAGCTCGAGCTCTA - 3' (SEQ ID NO:3) |
| Huntington's disease | Huntingtin | 5' - AAGGTTTTGTTAAAGGCCTTC - 3' (SEQ ID NO:4) |
| Huntington's disease | Huntingtin | 5' - CAGGAAATACATTTTCTTTGG - 3' (SEQ ID NO:5) |
| SCA1 | Ataxin-1 | 5' - AACCAAGAGCGGAGCAACGAA - 3' (SEQ ID NO:6) |
| SCA1 | Ataxin-1 | 5' - AACCAGTACGTCCACATTTCC - 3' (SEQ ID NO:7) |

Cell culture experiments have confirmed that each of the above sequences encodes an siRNA that is effective at suppressing the level of mRNA of the corresponding gene expressed by human cells.

It is important to note that the anti-ataxin-1 small interfering RNA, the anti-BACE1 small interfering RNA, and the anti-Huntington small interfering RNA illustrated here, as well as the other small interfering RNAs for treating neurodegenerative disorders, are just but some examples of the embodiment of the invention. Experimentation using neurosurgical methods with animals, known to those practiced in neuroscience, can be used to identify the candidate small interfering RNAs. The target site on the mRNA and the corresponding small interfering RNA identified by these empirical methods will be the one that will lead to the greatest-therapeutic effect when administered to patients with the subject neurodegenerative disease.

In reference to the nucleic acid molecules of the present invention, the small interfering RNA are targeted to complementary sequences in the mRNA sequence coding for the production of the target protein, either within the actual protein coding sequence, or in the 5-prime untranslated region or the 3-prime untranslated region. After hybridization, the host enzymes guided by the siRNA can cleave the mRNA sequence. Perfect or a very high degree of complementarity is needed for the small interfering RNA to be effective. A percent complementarity indicates the percentage of contiguous residues in a nucleic acid molecule that can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementarity, respectively). "Perfect complementarity" means that all the contiguous residues of a nucleic acid sequence will hydrogen bond with the same number of contiguous residues in a second nucleic acid sequence. However, it should be noted that single mismatches, or base-substitutions, within the siRNA sequence can substantially reduce the gene silencing activity of a small interfering RNA.

The small interfering RNA that target the specified sites in alpha-synuclein, BACE1 (including variants thereof, e.g. variants A, B, C, and D), huntingtin, ataxin-1, ataxin-3 and/or atrophin-1 RNAs represent a novel therapeutic approach to treat Parkinson's disease, Alzheimer's disease, Huntington's disease, Spinocerebellar 1, Spinocerebellar Ataxia Type 3, and/or dentatorubral-pallidoluysian atrophy in a cell or tissue.

In preferred embodiments of the present invention, a small interfering RNA is 15 to 30 nucleotides in length. In particular embodiments, the nucleic acid molecule is 15, 16, 17, 18, 19, 20, 21, 22, 23, 24, 25, 26, 27, 28, 29, or 30 nucleotides in length. In preferred embodiments the length of the siRNA sequence can be between 19-30 base pairs, and more preferably between 21 and 25 base pairs, and more preferably between 21 and 23 base pairs.

In a preferred embodiment, the invention provides a method for producing a class of nucleic acid-based gene inhibiting agents that exhibit a high degree of specificity for the RNA of a desired target. For example, the small interfering RNA is preferably targeted to a highly conserved sequence region of target RNAs encoding alpha-synuclein, BACE1 (including variants thereof, e.g. variants A, B, C, and D), huntingtin, ataxin-1, ataxin-3 and/or atrophin-1 RNA such that specific treatment of a disease or condition can be provided with either one or several nucleic acid molecules of the invention. Further, interfering RNA sequences can optionally be selected by identifying regions in the target sequence that begin with a pair of adenine bases (AA). SiRNAs can be constructed *in vitro* or *in vivo* using appropriate transcription enzymes or expression vectors.

The vector for delivery of foreign DNA to neurons in the brain is adeno-associated virus (AAV), such as recombinant adeno-associated virus serotype 2 or recombinant adeno-associated virus serotype 5. Alternatively, other viral vectors, such as herpes simplex virus, may be used for delivery of foreign DNA to central nervous system neurons.

SiRNAs can be constructed *in vitro* using DNA oligonucleotides. These oligonucleotides can be constructed to include an 8 base sequence complementary to the 5-prime end of a DNA-dependent RNA polymerase promoter (e.g., T7 promoter, T3 promoter, SP6 promoter) primer included in the SILENCER siRNA (Ambion Construction Kit 1620). Each gene specific oligonucleotide is annealed to a supplied T7 promoter primer, and a fill-in reaction with Klenow fragment generates a full-length DNA template for transcription into RNA. Two *in vitro* transcribed RNAs (one the antisense to the other) are generated by *in vitro* transcription reactions and then hybridized to each other to make double-stranded RNA. The double-stranded RNA product is treated with DNase (to remove the DNA transcription templates) and RNase (to polish the ends of the double-stranded RNA), and column purified to provide the siRNA that can be delivered and tested in cells.

Construction of siRNA vectors that express siRNAs within mammalian cells typically use an RNA polymerase III promoter to drive expression of a short hairpin RNA that mimics the structure of an siRNA. The insert that encodes this hairpin is designed to have two inverted repeats separated by a short spacer sequence. One inverted repeat is complementary to the mRNA to which the siRNA is targeted. A string of six consecutive thymidines added to the 3-prime end serves as a pol III transcription termination site. Once inside the cell, the vector constitutively expresses the hairpin RNA. The hairpin RNA is processed into an siRNA which induces silencing of the expression of the target gene, which is called RNA interference (RNAi).

Any suitable RNA polymerase III (pol III) promoter may be used in an siRNA expression vector to drive the expression of a small hairpin RNA. Exemplary promoters include, but are not limited to, well-characterized human and mouse U6 promoters and the human H1 promoter. RNA pol III was chosen to drive siRNA expression because it expresses relatively large amounts of small RNAs in mammalian cells and it terminates transcription upon incorporating a string of 3-6 uridines.

The polymerase chain reaction (PCR) used in the construction of siRNA expression plasmids and/or viral vectors is carried out in accordance with known techniques. *See, for example,* U.S. Patent Nos. 4,683,195 (Mullis et al.), 4,683,202 (Mullis), 4,800,159 (Mullis et al.), and 4,965,188 (Mullis et al.). In general, PCR involves a treatment of a nucleic acid sample (e.g., in the presence of a heat stable DNA polymerase) under hybridizing conditions, with one oligonucleotide primer for each strand of the specific sequence to be detected. An extension product of each primer which is synthesized is complementary to each of the two nucleic acid strands, with the primers sufficiently complementary to each strand of the specific sequence to hybridize therewith. The extension product synthesized from each primer can also serve as a template for further synthesis of extension products using the same primers. Following a sufficient number of rounds of synthesis of extension products, the sample is analyzed to assess whether the sequence or sequences to be detected are present. Detection of the amplified sequence may be carried out by visualization following EtBr staining of the DNA following gel electrophoresis, or using a detectable label in accordance with known techniques, and the like. For a review on PCR techniques (see PCR Protocols, A Guide to Methods and Amplifications, Michael et al. Eds, Acad. Press, 1990).

### NEUROLOGICAL DISEASES CAUSED BY THE ABSENCE OF A PROTEIN

Rare genetic disorders, known as inborn errors of metabolism, result in the inability of the body to turn food into energy (i.e., metabolize food) normally. The disorders are usually caused by defects in the enzymes involved in the biochemical pathways that break down food components. Further, lysosomal storage disorder (LSD) refers to an inherited disease characterized by a defect in the functional expression of any of the lysosomal enzymes.

Such diseases as inborn errors of metabolism may be treated by gene therapy. Specifically, the defective or missing gene may be identified by methods known to one of skill in the art, after which a replacement gene may be prepared and supplied to the missing site.

Exemplary polynucleotides encoding for deficient enzymes, and the disease associated with the deficient enzyme, are listed in Table 2.

**TABLE 2: Inborn errors of metabolism with neurological involvement, the enzyme deficiency causing each disease, and animal models associated with each**

| *Disease* | *Alternative Name* | *Enzyme Name Genbank Accession# (size of Genbank entry in base pairs)* | *Size of protein coding region* | *Neurological involvement* | *Online Mendelian Inheritance in Man (OMIM) Entry* | *Animal Models* |
|---|---|---|---|---|---|---|
| *Gangliosidosis (Sphingolipidosis)* | | | | | | |
| Gaucher's disease Types II / III | Gaucher's disease | beta-glucosidase (glucocerebrosidase) NM_000157 (2275 bp) | 1611 bp | Type II: dysphagia, palsy | Type II: 230900 | |
| | | | | Type III: ataxia, seizures, dementia | Type III: 231000 | |
| Sphingomyelin lipidosis | Niemann-Pick disease | acid sphingomyelinase NM_000543 (2373 bp) | 1890 bp | Hypotonia, spasticity, rigidity, mental retardation | 257200 | Mouse (ASM knock-out) |
| | Type A | | | | | |
| Globoid cell leukodystrophy | Krabbe's disease | galactocerebrosidase NM_000153 (3986 bp) | 2010 bp | cerebral atrophy, seizures | 245200 | Dogs (West Highland white terriers, and Cairn terriers) |
| Metachromatic leukodystrophy | Metachromatic leukodystrophy | arylsulfatase A NM_000487 (2039 bp) | 1524 bp | Rigidity, mental deterioration, convulsions; psychiatric symptoms in adult onset disease | 250100 | Mouse (ARSA knock-out) |
| Metachromatic leukodystrophy without arylsulfatase deficiency | Metachromatic leukodystrophy, variant form | saposin B NM_002778 (2767 bp) | 1575 bp | White matter lesions, cerebellar atrophy | 249900 | |
| Fabry's disease | Fabry's disease | alpha-galactosidase A NM_000169 (1350 bp) | 1290 bp | Autonomic dysfunction, neuropathic pain | 301500 | Mouse (GLA knock-out) |
| GM1-gangliosidosis | Landing's disease | beta-galactosidase NM_000404 (2409 bp) | 2034 bp | Severe cerebral degeneration | 230500 | Dogs (Portuguese water dogs) |
| GM2-gangliosidosis Type I | Tay-Sachs disease | beta-hexosaminidase A NM_000520 (2255 bp) | 1590 bp | Psychomotor degeneration, psychiatric symptoms | 272800 | Mouse (HEXA knock-out) |
| GM2-gangliosidosis Type II | Sandhoff's disease | beta-hexosaminidase A NM_000520 (2255bp) and beta-hexosaminidase B NM_000521 (1857 bp) | 1590 bp | Cerebellar ataxia, dysarthria | 268800 | Mouse (HEXB knock-out) |
| | | | 1671 bp | | | |

| *Glycoprotein disorders* | | | | | | |
|---|---|---|---|---|---|---|
| Fucosidosis | Fucosidosis | alpha-L-fucosidase NM_000147 (2035 bp) | 1386 bp | Mental retardation, cerebral atrophy, seizures | 230000 | Dog (English Springer spaniels) |
| alpha-Mannosidosis Types I / II | Mannosidosis | alpha-D-mannosidase NM_000528 (3443 bp) | 3033 bp | Mental retardation | 248500 | Cats, angus cattle, guinea pigs |
| beta-Mannosidosis | | beta-D-mannosidase NM_005908 (3308 bp) | 2640 bp | Hyperactivity, mental retardation | 248510 | Goats (see Leipprandt, 1996) |
| Aspartylglucosaminuria | Aspartylglucosa minuria | N-aspartyl-beta-glucosaminidase NM_000027 (2041 bp) | 1041 bp | 3^{rd} most common genetic cause of mental retardation | 208400 | Mouse (AGA knock-out) |

| *Glycogen storage diseases* | | | | | | |
|---|---|---|---|---|---|---|
| Glycogen storage disease Type II | Pompe's disease | alpha-glucosidase NM_000152 (3846 bp) | 2859 bp | Hypotonia | 232300 | Quails (Japanese) |
| Glycogen storage disease Type IIb | Danon disease | LAMP-2 NM_013995 (4006 bp) | 1233 bp | Mental retardation, to variable degrees | 300257 | Mouse (LAMP2 knock-out, Tanaka, 2000) |
| Glycogen storage disease Type IV | Andersen's disease | glycogen branching enzyme NM_000158 (2913 bp) | 2109 bp | Variable | 232500 | |

| *Mucolipidosis* | | | | | | |
|---|---|---|---|---|---|---|
| Mucolipidosis Type I | Sialidosis Type II | neuraminidase NM_000434 (1943 bp) | 1248 bp | Hypotonia, ataxia, seizures | 256550 | Mouse (NEU1 knock-out) |
| Mucolipidosis Type II / III | 1-cell disease | phosphotransferase NM_032520 (1228 bp) | 918 bp | Severe psychomotor retardation | 252500 | Cat |

| *Mucopolysaccharidosis* | | | | | | |
|---|---|---|---|---|---|---|
| Mucopolysaccharidosis Type I | Hurler's syndrome, Scheie's syndrome | alpha-L-iduronidase NM_000203 (2197 bp) | 1962 bp | Mental retardation | 607014 | Cat, Dog |
| Mucopolysaccharidosis Type II | Hunter's syndrome | iduronate-2-sulfatase NM_000202 (2504 bp) | 1653 bp | Hydrocephalus, mental retardation, seizures | 309900 | Dog (Labrador retriever) |
| Mucopolysaccharidosis Type IIIA | Sanfilippo's syndrome | heparan-N-sulfatase NM_000199 (2740 bp) | 1509 bp | Hyperactivity, mental retardation, seizures, sleep disturbances | 252900 | Dog (wire-haired Dachshund) |
| Mucopolysaccharidosis Type IIIB | Sanfilippo's syndrome | alpha-N-acetylglucosaminidas e NM_000263 (2819 bp) | 2232 bp | | 252920 | Mouse (Naglu knock-out) |
| Mucopolysaccharidosis Type IIIC | Sanfilippo's syndrome | acetylCoA:N-acetyltransferase (specific gene still unknown) | | | 252930 | |
| Mucopolysaccharidosis Type IIID | Sanfilippo's syndrome | N-acetylglucosamine 6-sulfatase NM_002076 (5130 bp) | 1658 bp | | 252940 | Goat |
| Mucopolysaccharidosis Type IVA | Morquio syndrome | galactose 6-sulfatase NM_000512 (2328 bp) | 1569 bp | Cervical myelopathy | 253000 | |
| Mucopolysaccharidosis Type IVB | Morquio syndrome | beta-galactosidase NM_000404 (2409 bp) | 2034 bp | | 253010 | |
| Mucopolysaccharidosis Type VI | Maroteaux-Lamy syndrome | N-acetylgalactosamine 4-sulfatase NM_000046 (6089 bp) | 1601 bp | Cervical myelopathy, hydrocephalus | 253200 | Cat (Siamese) |
| Mucopolysaccharidosis Type VII | Sly syndrome | beta-glucuronidase NM_000181 (2191 bp) | 1956 bp | Mental retardation, hydrocephalus, neurodegeneration | 253220 | Cat, Dog |

| *Other Lysosomal Storage Disorders* | | | | | | |
|---|---|---|---|---|---|---|
| Cholesterol ester storage disease | Wolman disease | lysosomal acid lipase (acid cholesteryl ester hydrolase) NM_000235 (2493 bp) | 1200 bp | lipid accumulation in glia | 278000 | Mouse (LAL knock-out) |
| Farber lipogranulomatosis | Farber disease | acid ceramidase NM_004315 (2503 bp) | 1236 bp | mental retardation, seizures, cerebral atrophy | 228000 | |
| Galactosialidosis Types I / II | Schindler disease | N-acetyl-alpha-D-galactosaminidase NM_000262 (3598 bp) | 1236 bp | mental retardation, seizures | 104170 | |
| Neuronal ceroid lipofuscinosis (CLN1) | Batten disease | palmitoyl protein thioesterase NM_000310 (2279 bp) | 921 bp | Most common neurodegenerative disease in children; dementia, seizures | 600722 | Mouse (PPT1 knock-out, PPT2 knock-out) |
| Aspartoacylase deficiency | Canavan disease | aspartoacylase NM_000049 (1435 bp) | 942 bp | hypotonia, demyelination, severe mental defect | 271900 | |

The polynucleotides encoding for deficient enzymes listed in Table 2 are examples of polynucleotides that can be included in artificial AAV vectors of the present invention. However, the polynucleotides listed in Table 2 are not intended to be limiting, as one of skill in the art could identify additional diseases caused by the absence of a protein, and corresponding, could identify additional polynucleotides encoding deficient enzymes.

Further, one of skill in the art would recognize that polynucleotides homologous to those listed herein (e.g., in Table 2) can be included in artificial AAV vectors of the present invention. For example, polynucleotides with coding regions sharing a significant level of primary structure (e.g., a significant level of identity) with the coding regions present in the polynucleotides listed in Table 2 can be used. The level of identity is determined by aligning the two nucleotide sequences such that the residues that encode the putative active site of the encoded protein are in register, then further aligned to maximize the number of nucleotides that they have in common along the lengths of their sequences; gaps in either or both sequences are permitted in making the alignment in order to place the residues that encode the putative active site of the encoded protein in register and to maximize the number of shared nucleotides, although the nucleotides in each sequence must nonetheless remain in their proper order. Preferably, two nucleotide sequences are compared using the blastn program of the BLAST search algorithm, which is described by Altshul et al., (Nucl. Acids Res., 25, 3389-3402 (1997)), and available at the National Center for Biotechnology Information (e.g., on the World
Wide Web at ncbi.nlm.nih.gov/BLAST/). Preferably, the default values for all BLAST search parameters are used. In the comparison of two nucleotide sequences using the BLAST search algorithm, structural similarity is referred to as "identities." Preferably, two nucleotide acid sequences have, in increasing order of preference, preferably at least 70%, 80%, 90%, 91%, 92%, 93%, 94%, 95%, 96%, 97%, 98%, and 99% identity.

### ARTIFICIAL AAV VECTOR

An artificial AAV vector includes DNA encoding a biologically active agent, and can be used to deliver a gene or a gene-suppressing agent to a patient's neurons. Thus, the artificial AAV preferably includes a cassette to deliver a gene, or a cassette to deliver a gene-suppressing agent. For example, in the case of a gene therapy intended to supply a missing gene to the patient's brain, the expression cassette can include a promoter element, the coding sequences for the missing gene, and a polyadenylation signal sequence. For another example, in the case of a gene suppression therapy intended to suppress the expression of an endogenous gene in the patient's brain, the expression cassette can include a promoter element, the coding sequence for a small, interfering RNA (siRNA), and a termination sequence.

In one example, the artificial AAV vector is a double stranded vector. The double stranded vector, which may include either type of expression cassette, includes a 5-prime copy of the inverted terminal repeat (AAV-ITR) from the adeno-associated virus genome, followed by an expression cassette for a gene or gene-suppressing agent (whose identity depends upon the neurological disorder to be treated), followed at the 3-prime end by a 3-prime copy of the AAV-ITR.

In another embodiment, the artificial AAV vector, which may include either type of expression cassette, is a single stranded vector. The single stranded vector includes a single stranded DNA segment including a 5-prime copy of the inverted terminal repeat (AAV-ITR) from the adeno-associated virus genome, followed by an expression cassette for a gene or gene-suppressing agent (whose identity depends upon the neurological disorder to be treated), followed at the 3-prime end by a 3-prime copy of the AAV-ITR. Optionally and preferably, the entire DNA sequence including either type of expression cassette is repeated in reverse complement order, so that the DNA sequence includes the 5-prime AAV-ITR, the expression cassette, an internal AAV-ITR, the reverse complement of the expression cassette, and the 3-prime AAV-ITR. The 3-prime AAV-ITR is the reverse complement of the 5-prime AAV-ITR (as illustrated, for example, in Example 1 herein), and either a 3-prime or 5-prime AAV-ITR can be used as the internal AAV-ITR. The resulting "self-complementary" artificial AAV vector is preferred because it may produce more effective transfection of neurons by the DNA. *See, for example,* Fu et al., Molecular Therapy 8:911-917 (2003).

It will be appreciated by those skilled in the art that the embodiment of a double-stranded artificial AAV vector and the embodiment of a single-stranded self-complementary artificial AAV vector differ only in that the single stranded self-complementary vector has a single, single-stranded AAV-TTR joining the complementary strands of the expression cassette (covalently joining the 3-prime end of one strand to the 5-prime end of the complementary strand, as shown schematically in Figure 2) so that the entire artificial AAV vector is one single DNA strand "folded back" on itself with hydrogen bonds between the complementary strands of the expression cassette. In the case of the double stranded artificial AAV vector, there are double-stranded AAV-ITRs at the 5-prime end and the 3-prime end of the expression cassette with no covalent bond joining strands at either end (as illustrated schematically in Figure 3).

An exemplary method for preparing a double-stranded artificial AAV vector is disclosed. The method includes the steps of: assembling the 5-prime AAV-ITR, expression cassette, and 3-prime AAV-ITR in any suitable DNA plasmid using standard DNA cloning methods; liberating the 5-prime AAV-ITR, expression cassette, and 3-prime AAV-ITR from the plasmid by digesting the plasmid with a restriction enzyme that cuts the DNA at a site just 5-prime to the 5-prime AAV-ITR and just 3-prime to the 3-prime AAV-ITR; and purifying the linear DNA fragment consisting of the 5-prime AAV-ITR, expression cassette, and 3-prime AAV-ITR using standard methods. Optionally, the resulting linear double-stranded artificial AAV vector may be further processed by a thermal treatment step including, for example, heating the purified linear DNA fragment (e.g., heating to 65°C or higher for 10 minutes or more), followed by cooling (e.g., allowing the DNA fragment to cool slowly to room temperature over a period of 10 minutes or more). These heating and cooling steps can allow the AAV ITRs to assume a secondary structure, conducive to long-term gene expression from this double-stranded artificial AAV vector, as illustrated schematically in Figure 4.

Exemplary methods for preparing a single-stranded DNA as described herein above are also disclosed. One method includes the steps of: assembling the 5-prime AAV-ITR, expression cassette, and 3-prime AAV-ITR in any suitable DNA plasmid using standard DNA cloning methods; generating a single-stranded RNA transcript of the desired single-stranded DNA from the DNA plasmid using standard *in vitro* transcription methods; generating single-stranded DNA from the RNA transcript by reverse transcription using standard reverse transcription reaction methods; removing the RNA transcript from the reaction products by digestion of the RNA using RNase enzyme; and purifying the resulting single-stranded DNA product from the reaction products by standard DNA purification methods, such as gel purification or column affinity methods.

Another method includes the steps of: assembling the 5-prime AAV-ITR, expression cassette, and 3-prime AAV-ITR in any suitable DNA plasmid using standard DNA cloning methods; linearizing the circular plasmid by digesting the plasmid with a restriction enzyme that cuts the DNA at a single, known location in the plasmid sequence just 5-prime to the 5-prime AAV-ITR; chemically conjugating an affinity tag (e.g., a biotin molecule) to the 5-prime ends of each strand of the linearized plasmid; cutting the DNA sequence with a restriction enzyme that cuts the DNA at a second single, known location in the plasmid sequence just 3-prime to the 3-prime AAV-ITR, such that the restriction digest results in two linear double-stranded DNA segments of different sizes; separating the populations of DNA molecules by size using any suitable size separation method (e.g., column filtration or gel electrophoresis) and recovering the desired double-stranded DNA; and melting the DNA to separate its two complementary strands into two single strands and passing the mixture through an affinity column for the tag (e.g., a streptavidin affinity column when a biotin molecule is used as the affinity tag) such that the strand which was tagged in step 3 is captured on the column while the non-tagged single-strand flows through as the desired final product. This method can be advantageous for not involving any DNA or RNA polymerization steps that might introduce sequence errors in the final product.

In the case of a self-complementary AAV, the method includes the steps of: assembling the 5-prime AAV-ITR, expression cassette, internal AAV-ITR, reverse complement of the same expression cassette, and 3-prime AAV-ITR into any suitable DNA plasmid using standard DNA cloning methods; linearizing the circular plasmid by digesting the plasmid with restriction enzymes that cut out the desired DNA sequence (from the 5-prime AAV-ITR through the 3-prime AAV-ITR); recovering the desired DNA sequence from step 2 by size using any suitable size separation method; melting this double-stranded DNA to separate its two complementary strands into two single strands; and lowering the temperature (preferably slowly) of the melted DNA to allow the single strands to self-anneal into a hairpin form. All of the resulting single strands ("sense" or "anti-sense" strand) would be useful as the final product, since either strand would contain a copy of the desired expression cassette in a 5-prime to 3-prime orientation.

### COMPOSITIONS

For embodiments in which the composition is delivered across the blood-brain barrier, the composition includes, for example, a liposome as described, for example, in U.S. Patent No. 6,372,250 (Pardridge), and a pharmaceutically acceptable carrier. Preferably the liposome is a receptor-specific liposome, wherein the receptor-specific'liposome includes: a liposome having an exterior surface and an internal compartment; an artificial adeno-associated virus (AAV) vector located within the internal compartment of the liposome; one or more blood-brain barrier and brain cell membrane targeting agents; and one or more conjugation agents (e.g., polyethylene glycol (PEG) strands), wherein each targeting agent is connected to the exterior surface of the liposome via at least one of the conjugation agents. Receptor-specific liposomes including an artificial adeno-associated virus (AAV) vector located within the internal compartment of the liposome can be prepared by the general methods described in U.S. Patent No. 6,372,250 (Pardridge), except that the artificial adeno-associated virus (AAV) vector is used instead of the plasmid DNA.

As used herein, a "targeting agent" refers to a chemical species that interacts, either directly or indirectly, with the surface of a cell, for example, with a molecule present on the surface of a cell, e.g., a receptor. The interaction can be, for example, an ionic bond, a hydrogen bond, a Van der Waals force, or a combination thereof. Examples of targeting agents include, for example, saccharides, polypeptides (including hormones), polynucleotides, fatty acids, and catecholamines. As used herein, the term "saccharide" refers to a single carbohydrate monomer, for example, glucose, or two or more covalently bound carbohydrate monomers, i.e., an oligosaccharide. An oligosaccharide including 4 or more carbohydrate monomers can be linear or branched. Examples of oligosaccharides include lactose, maltose, and mannose. As used herein, "polypeptide" refers to a polymer of amino acids and does not refer to a specific length of a polymer of amino acids. Thus, for example, the terms peptide, oligopeptide, protein, antibody, and enzyme are included within the definition of polypeptide. This term also includes post-expression modifications of the polypeptide, for example, glycosylations (e.g., the addition of a saccharide), acetylations, phosphorylations and the like.

Liposomes as described herein can deliver biologically active agents across the blood-brain barrier, followed by expression in the brain. Liposomes and nanoparticles are exemplary forms of nanocontainers that are commonly used for encapsulation of drugs. The liposomes preferably have diameters of less than 200 nanometers. Liposomes having diameters of between 50 and 150 nanometers are preferred. Especially preferred are liposomes or other nanocontainers having external diameters of about 80 nanometers. Suitable types of liposomes are made with neutral phospholipids such as 1-palmitoyl-2-oleoyl-sn-glycerol-3-phosphocholine (POPC), diphosphatidyl phosphocholine, distearoylphosphatidylethanolamine (DSPE), or cholesterol, along with a small amount (1%) of cationic lipid, such as didodecyldimethylammonium bromide (DDAB) to stabilize the DNA within the liposome.

Although the invention has been described using liposomes as the preferred nanocontainer, it will be recognized by those skilled in the art that other nanocontainers may be used. For example, the liposome can be replaced with a nanoparticle or any other molecular nanocontainer with a diameter <200 nm that can encapsulate the DNA and protect the nucleic acid from nucleases while the formulation is still in the blood or in transit from the blood to the intracellular compartment of the target cell. Also, instead of using conjugation agents such as PEG strands, one or more other polymeric substances, such as sphingomylein, can be attached to the surface of the liposome or nanocontainer and serve the dual purpose of providing a scaffold for conjugation of the "transportable peptide" and for delaying the removal of the formulation from blood and optimizing the plasma pharmacokinetics. Further, the present invention contemplates delivery of DNA to any group of cells or organs which have specific target receptors. The liposomes may be used to deliver DNA to organs, such as liver, lung and spleen.

The liposomes may be combined with any suitable pharmaceutical carrier for intravenous administration. Intravenous administration of the composition is the preferred route since it is the least invasive. Other routes of administration are possible, if desired. Suitable pharmaceutically acceptable carriers include saline, Tris buffer, phosphate buffer, or any other aqueous solution. An appropriate dosage can be established by procedures well known to those of ordinary skill in the art.

Those of skill in the art are familiar with the principles and procedures discussed in widely known and available sources as Remington's Pharmaceutical Science (17th Ed., Mack Publishing Co., Easton, PA, 1985) and Goodman and Gilman's The Pharmaceutical Basis of Therapeutics (8th Ed., Pergamon Press, Elmsford, NY, 1990).

In a preferred embodiment of the present invention, the compositions or precursors or derivatives thereof are formulated in accordance with standard procedure as a pharmaceutical composition adapted for delivered administration to human beings and other mammals. Typically, compositions for intravenous administration are solutions in sterile isotonic aqueous buffer.

Where necessary, the composition may also include a solubilizing agent and a local anesthetic to ameliorate any pain at the site of the injection. Generally, the ingredients are supplied either separately or mixed together in unit dosage form, for example, as a dry lyophilized powder or water free concentrate in a hermetically sealed container such as an ampule or sachette indicating the quantity of active agent. Where the composition is to be administered by infusion, it can be dispensed with an infusion bottle containing sterile pharmaceutical grade water or saline. Where the composition is administered by injection, an ampule of sterile water for injection or saline can be provided so that the ingredients may be mixed prior to administration.

In cases other than intravenous administration, the composition can contain minor amounts of wetting or emulsifying agents, or pH buffering agents. The composition can be a liquid solution, suspension, emulsion, gel, polymer, or sustained release formulation. The composition can be formulated with traditional binders and carriers, as would be known in the art. Formulations can include standard carriers such as pharmaceutical grades of mannitol, lactose, starch, magnesium stearate, sodium saccharide, cellulose, magnesium carbonate, etc., inert carriers having well established functionality in the manufacture of pharmaceuticals. Various delivery systems are known and can be used to administer a composition of the present invention including encapsulation in liposomes, microparticles, microcapsules and the like.

In yet another preferred embodiment, compositions can be formulated as neutral or salt forms. Pharmaceutically acceptable salts include those formed with free amino groups such as those derived from hydrochloric, phosphoric, acetic, oxalic, tartaric acids and the like, and those formed with free carboxyl groups such as those derived from sodium, potassium, ammonium, calcium, ferric hydroxides, isopropylamine, thriethylamine, 2-ethylamino ethanol, histidine, procaine or similar.

The amount of a composition of the present invention which will be effective in the treatment of a particular disorder or condition will depend on the nature of the disorder or condition, and can be determined by standard clinical techniques, well established in the administration of compositions. The precise dose to be employed in the formulation will also depend on the route of administration, and the seriousness of the disease or disorder, and should be decided according to the judgment of the practitioner and the patient's needs. Suitable dose ranges for intracranial administration are generally about 10³ to 10¹⁵ infectious units of viral vector per microliter delivered in 1 to 3000 microliters of single injection volume. Addition amounts of infections units of vector per micro liter would generally contain about 10⁴, 10⁵, 10⁶, 10⁷, 10⁸, 10⁹, 10¹⁰, 10¹¹, 10¹², 10¹³, 10¹⁴ infectious units of viral vector delivered in about 10, 50, 100, 200, 500, 1000, or 2000 microliters. Appropriate dosage may be extrapolated from dose-responsive curves derived from *in vitro* or *in vivo* test systems.

Unless defined otherwise, the scientific and technological terms and nomenclature used herein have the same meaning as commonly understood by a person of ordinary skill to which this invention pertains. Generally, the procedures for cell cultures, infection, molecular biology methods and the like are common methods used in the art. Such standard techniques can be found in reference manuals such as for example Sambrook et al. (1989, Molecular Cloning - A Laboratory Manual, Cold Spring Harbor. Laboratories) and Ausubel et al. (1994, Current Protocols in Molecular Biology, Wiley, New York).

The present invention is illustrated by the following examples.

### EXAMPLES

### EXAMPLE 1:

Applicants have developed the DNA coding for an siRNA that suppresses expression of beta-amyloid cleaving enzyme (BACE1). Suppression of BACE1 in neuronal cells is known to abolish the production of beta-amyloid fragments from amyloid-precursor protein. Soluble beta-amyloid fragments are believed to be the cause of cognitive dysfunction and neurodegeneration in Alzheimer's disease, and insoluble aggregations of these fragments are known to be contained in the characteristic plaques in the Alzheimer's brain. Expression cassettes of the DNA coding for the anti-BACE1 siRNA were engineered into constructs that are flanked by AAV-ITRs. In the embodiment illustrated herein, the ITRs are from AAV2. However it would be clear to one of skill in the art that ITRs from other serotypes could also be used. Data showing that this anti-BACE1 siRNA does, in fact, result in reduced expression of BACE1 messenger RNA and reduced activity of BACE1 enzyme in cell cultures has been obtained. (See Examples 2-4, herein below).

Following the disclosed methods, AAV-ITR flanked DNA encoding for anti-BACE1 siRNA can be packaged inside liposomes formulated for transport across the blood-brain barrier and into brain cells. The resulting composition would be delivered via a neurovascular catheter to one or more of the blood vessels supplying an Alzheimer patient's brain. Delivery of this DNA to the patient's brain is expected to result in reduction of the expression of BACE1 enzyme throughout the patient's brain for a period of many years. Although the clearance mechanisms that the brain has for ridding itself of beta-amyloid and beta-amyloid plaques are evidently inadequate to prevent Alzheimer's disease, it is expected that by reducing the production of beta-amyloid "at the source," the clearance mechanisms will be better able to reduce the effects that beta-amyloid has had on the patient's brain. As a result, it is hypothesized that the progression of the disease will be slowed or arrested, and the patient will stabilize and perhaps improve.

Thus, delivery of this DNA via the neurovasculature throughout the brain can provide a means for treating Alzheimer's disease in patients. If the therapy proves to be safe and without adverse side-effects, it might also have potential as a preventative treatment. That is, it might be used to prophylactically "inoculate" all aging persons in the population against possible future development of Alzheimer's disease.

Various embodiments of the synthetic AAV DNA sequence for the biological component of the present invention as it pertains to a therapy for Alzheimer's disease are as follows. "AAV-U6-MB1749" (SEQ ID NO:8) is the sequence for a synthetic AAV containing the DNA code for MB1749, which is an siRNA that is effective for suppressing the expression of beta-amyloid cleaving enzyme type 1 (BACE1) as a treatment for Alzheimer's disease:

**TABLE 3: SEQ ID NO:8: "AAV-U6-MB 1749"**

| *Positions* | *Description* | *DNA Sequence* |
|---|---|---|
| 1-145 | 5-prime AAV-ITR | 5' - TTGGCCACTC CCTCTCTGCG CGCTCGCTCG CTCACTGAGG CCGGGCGACC AAAGGTCGCC CGACGCCCGG GCTTTGCCCG GGCGGCCTCA GTGAGCGAGC GAGCGCGCAG AGAGGGAGTG GCCAACTCCA TCACTAGGGG TTCCT |
| | | GAAT |
| 150-482 | Human U6 RNA polymerase III promoter | TCCCCAGTGG AAAGACGCGC AGGCAAAACG CACCACGTGA CGGAGCGTGA CCGCGCGCCG AGCGCGCGCC AAGGTCGGGC AGGAAGAGGG CCTATTTCCC ATGATTCCTT CATATTTGCA TATACGATAC AAGGCTGTTA GAGAGATAAT TAGAATTAAT TTGACTGTAA ACACAAAGAT ATTAGTACAA AATACGTGAC GTAGAAAGTA ATAATTTCTT GGGTAGTTTG CAGTTTTAAA ATTATGTTTT AAAATGGACT ATCATATGCT TACCGTAACT TGAAAGTATT TCGATTTCTT GGGTTTATAT ATCTTGTGGA AAGGACGCGG GAT |
| 483-485 | provides BamHI restriction site GGATCC | CCG |
| 486-506 | MB1749 siRNA | AAGACTGTGGCTACAACATTC |
| 507-515 | loop | TTCAAGAGA |
| 516-537 | reverse complement of MB1749 | GAATGTTGTAGCCACAGTCTTC |
| 538-543 | Terminator sequence for RNA polymerase III | TTTTTT |
| 544-547 | | GGAA |
| 548-553 | HindIII restriction site | AAGCTT |
| 554-698 | 3-prime AAV-ITR | AGGAACCCCT AGTGATGGAG TTGGCCACTC CCTCTCTGCG CGCTCGCTCG CTCACTGAGG CCGGGCGACC AAAGGTCGCC CGACGCCCGG GCTTTGCCCG GGCGGCCTCA GTGAGCGAGC GAGCGCGCAG AGAGGGAGTG GCCAA - 3' |

"AAV-H1-MB1749" (SEQ ID NO:9) is the sequence for a synthetic AAV containing the DNA code for MB 1749 with expression driven by the human H1 RNA polymerase III promoter, rather than the U6 promoter:

**TABLE 4: SEQ ID NO:9: "AAV-H1-MB1749"**

| *Positions* | *Description* | *DNA Sequence* |
|---|---|---|
| 1-145 | 5-prime AAV-ITR | 5' - TTGGCCACTC CCTCTCTGCG CGCTCGCTCG CTCACTGAGG CCGGGCGACC AAAGGTCGCC CGACGCCCGG GCTTTGCCCG GGCGGCCTCA GTGAGCGAGC GAGCGCGCAG AGAGGGAGTG GCCAACTCCA TCACTAGGGG TTCCT |
| 146 | | G |
| 147-246 | Human H1 RNA polymerase III promoter | AATTCATATT TGCATGTCGC TATGTGTTCT GGGAAATCAC CATAAACGTG AAATGTCTTT GGATTTGGGA ATCTTATAAG TTCTGTATGA GACCACTCGG |
| 247-251 | provides BamHI restriction site GGATCC | ATCCG |
| 252-272 | MB1749 siRNA | AAGACTGTGGCTACAACATTC |
| 273-281 | loop | TTCAAGAGA |
| 282-303 | reverse complement of MB1749 | GAATGTTGTAGCCACAGTCTTC |
| 304-309 | Terminator sequence for RNA polymerase III | TTTTTT |
| 310-313 | | GGAA |
| 314-319 | HindIII restriction site | AAGCTT |
| 320-464 | 3-prime AAV-ITR | AGGAACCCCT AGTGATGGAG TTGGCCACTC CCTCTCTGCG CGCTCGCTCG CTCACTGAGG CCGGGCGACC AAAGGTCGCC CGACGCCCGG GCTTTGCCCG GGCGGCCTCA GTGAGCGAGC GAGCGCGCAG AGAGGGAGTG GCCAA - 3' |

"SC-AAV-U6-MB 1749" (SEQ ID NO:10) is the sequence for a synthetic self-complementary AAV containing the DNA code for MB1749 with expression driven by the human U6 RNA polymerase III promoter:

**TABLE 5: SEQ ID NO:10: "SC-AAV-U6-MB 1749"**

| Positions | Description | DNA Sequence |
|---|---|---|
| 1-145 | 5-prime AAV-ITR | 5′ - TTGGCCACTC CCTCTCTGCG CGCTCGCTCG CTCACTGAGG CCGGGCGACC AAAGGTCGCC CGACGCCCGG GCTTTGCCCG GGCGGCCTCA GTGAGCGAGC GAGCGCGCAG AGAGGGAGTG GCCAACTCCA TCACTAGGGG TTCCT |
| | | GAAT |
| 150-482 | Human U6 RNA polymerase III promoter | TCCCCAGTGG AAAGACGCGC AGGCAAAACG CACCACGTGA CGGAGCGTGA CCGCGCGCCG AGCGCGCGCC AAGGTCGGGC AGGAAGAGGG CCTATTTCCC ATGATTCCTT CATATTTGCA TATACGATAC AAGGCTGTTA GAGAGATAAT TAGAATTAAT TTGACTGTAA ACACAAAGAT ATTAGTACAA AATACGTGAC GTAGAAAGTA ATAATTTCTT GGGTAGTTTG CAGTTTTAAA ATTATGTTTT AAAATGGACT ATCATATGCT TACCGTAACT TGAAAGTATT TCGATTTCTT GGGTTTATAT ATCTTGTGGA AAGGACGCGG GAT |
| 483-485 | provides BamHI restriction site GGATCC | CCG |
| 486-506 | MB1749 siRNA | AAGACTGTGGCTACAACATTC |
| 507-515 | loop | TTCAAGAGA |
| 516-537 | reverse complement of MB1749 | GAATGTTGTAGCCACAGTCTTC |
| 538-543 | Terminator sequence for RNA polymerase III | TTTTTT |
| 544-547 | | GGAA |
| 548-553 | HindIII restriction site | AAGCTT |
| 554-698 | Internal AAV-ITR | AGGAACCCCT AGTGATGGAG TTGGCCACTC CCTCTCTGCG CGCTCGCTCG CTCACTGAGG CCGGGCGACC AAAGGTCGCC CGACGCCCGG GCTTTGCCCG GGCGGCCTCA GTGAGCGAGC GAGCGCGCAG AGAGGGAGTG GCCAA |
| 699-1126 | Reverse complement of 573 down to 146 | CTCCATCACT AGGGGTTCCT AAGCTTTTCC AAAAAAGAAG ACTGTGGCTA CAACATTCTC TCTTGAAGAA TGTTGTAGCC ACAGTCTTCG GATCCCGCGT CCTTTCCACA AGATATATAA ACCCAAGAAA TCGAAATACT TTCAAGTTAC GGTAAGCATA TGATAGTCCA TTTTAAAACA TAATTTTAAA ACTGCAAACT ACCCAAGAAA TTATTACTTT CTACGTCACG TATTTTGTAC TAATATCTTT GTGTTTACAG TCAAATTAAT TCTAATTATC TCTCTAACAG CCTTGTATCG TATATGCAAA TATGAAGGAA TCATGGAAA TAGGCCCTCT TCCTGCCCGA CCTTGGCGCG CGCTCGGCGC GCGGTCACGC TCCGTCACGT GGTGCGTTTT GCCTGCGCGT CTTTCCACTG GGGAATTC |
| 1127-1271 | 3-prime AAV-ITR | AGGAACCCCT AGTGATGGAG TTGGCCACTC CCTCTCTGCG CGCTCGCTCG CTCACTGAGG CCGCCCGGGC AAAGCCCGGG CGTCGGGCGA CCTTTGGTCG CCCGGCCTCA GTGAGCGAGC GAGCGCGCAG AGAGGGAGTG GCCAA - 3' |

"SC-AAV-H1-MB1749" (SEQ ID NO:11) is the sequence for a synthetic self-complementary AAV containing the DNA code for MB 1749 with expression driven by the human H1 RNA polymerase III promoter:

**TABLE 6: SEQ ID NO:11: "SC-AAV-H1-MB1749"**

| *Positions* | *Description* | *DNA Sequence* |
|---|---|---|
| 1-145 | 5-prime AAV-ITR | 5' - TTGGCCACTC CCTCTCTGCG CGCTCGCTCG CTCACTGAGG CCGGGCGACC AAAGGTCGCC CGACGCCCGG GCTTTGCCCG GGCGGCCTCA GTGAGCGAGC GAGCGCGCAG AGAGGGAGTG GCCAACTCCA TCACTAGGGG TTCCT |
| 146 | | G |
| 147-246 | Human H1 RNA polymerase III promoter | AATTCATATT TGCATGTCGC TATGTGTTCT GGGAAATCAC CATAAACGTG AAATGTCTTT GGATTTGGGA ATCTTATAAG TTCTGTATGA GACCACTCGG |
| 247-251 | provides BamHI restriction site GGATCC | ATCCG |
| 252-272 | MB1749 siRNA | AAGACTGTGGCTACAACATTC |
| 273-281 | loop | TTCAAGAGA |
| 282-303 | reverse complement of MB1749 | GAATGTTGTAGCCACAGTCTTC |
| 304-309 | Terminator sequence for RNA polymerase III | TTTTTT |
| 310-313 | | GGAA |
| 314-19 | HindIII restriction site | AAGCTT |
| 320-464 | Internal AAV-ITR | AGGAACCCCT AGTGATGGAG TTGGCCACTC CCTCTCTGCG CGCTCGCTCG CTCACTGAGG CCGGGCGACC AAAGGTCGCC CGACGCCCGG GCTTTGCCCG GGCGGCCTCA GTGAGCGAGC GAGCGCGCAG AGAGGGAGTG GCCAA |
| 465-658 | Reverse complement of 339 down to 146 | CTCCATCACT AGGGGTTCCT AAGCTTTTCC AAAAAAGAAG ACTGTGGCTA CAACATTCTC TCTTGAAGAA TGTTGTAGCC ACAGTCTTCG GATCCGAGTG GTCTCATACA GAACTTATAA GATTCCCAAA TCCAAAGACA TTTCACGTTT ATGGTGATTT CCCAGAACAC ATAGCGACAT GCAAATATGA ATTC |
| 659-803 | 3-prime AAV-ITR | AGGAACCCCT AGTGATGGAG TTGGCCACTC CCTCTCTGCG CGCTCGCTCG CTCACTGAGG CCGCCCGGGC AAAGCCCGGG CGTCGGGCGA CCTTTGGTCG CCCGGCCTCA GTGAGCGAGC GAGCGCGCAG AGAGGGAGTG GCCAA - 3' |

AAV-U6-MB1749 DNA construct (SEQ ID NO:8) has been produced and biologically packaged into AAV virus particles, and stereotactically injected into the brains of transgenic mice that harbor the human gene for amyloid-precursor protein. The viral-mediated gene therapy may reduce the manifestations of the Alzheimer's-like disease in these mice.

### EXAMPLE 2: Suppression of BACE1 mRNA in vitro using siRNA.

An siRNA sequence which effectively suppresses the expression of BACE1 messenger RNA *in vitro* in the Neuro2a mouse neuronal cell line has been identified and designated as MB 1749. MB 1749 refers to "mouse Bace1 sequence position 1749" and corresponds to nucleotides 1749 through 1769 in the mouse BACE1 cDNA sequence (Genbank accession number NM_011792.2). The nucleotide sequence is as follows:
SEQ ID NO:12: 5'- AAGACTGTGGCTACAACATTC - 3'

This sequence is identical (100% homologous) with all four variants of the human cDNA sequence for BACE1 (A, B, C, and D), and is found at the following positions in the various human BACE1 cDNA sequences in Genbank:

**TABLE 7: Human cDNA Variants**

| *Name* | *Accession Number* | *Start Position* | *End Position* |
|---|---|---|---|
| Homo sapiens BACE transcript variant A | NM_012104.2 | 1768 | 1788 |
| Homo sapiens BACE transcript variant B | NM_138972.1 | 1693 | 1713 |
| Homo sapiens BACE transcript variant C | NM_138971.1 | 1636 | 1656 |
| Homo sapiens BACE transcript variant D | NM_138973.1 | 1561 | 1581 |

As part of selecting the MB1749 sequence as a candidate siRNA for silencing BACE1 expression, the sequence was compared to all other known genomic DNA sequences for *Homo sapiens* using the BLAST software provided by the National Institutes of Health National Center for Biotechnology Information on the world wide web at the BLAST site located at ncbi.nlm.nih dot gov, and it was found not to be homologous to any other known human gene sequence.

For purposes of testing with Neuro2a cell cultures, double-stranded MB1749 siRNA was constructed using an *in vitro* transcription method. The following two synthetic DNA oligonucleotides were obtained from MWG-Biotech, Inc. (a DNA synthesis service):
SEQ ID NO:13: 5' - AA GACTGTGGCTACAACATTC CCTGTCTC - 3'
SEQ ID NO:14: 5' - AA GAATGTTGTAGCCACAGTC CCTGTCTC - 3'

The siRNA was produced from these DNA oligonucleotides using reagents commercially provided by Ambion, Inc. (Silencer siRNA Construction Kit, Catalog Number 1620) following the manufacturer's protocol, as follows: The supplied T7 promoter primers were hybridized to each of the two above DNA oligonucleotide transcription templates. The 3-prime ends of the hybridized oligo were extended by the Klenow fragment of DNA polymerase to create double-stranded siRNA transcription templates. The sense and antisense siRNA templates were transcribed by T7 RNA polymerase and the resulting RNA transcript were hybridized to create double-stranded RNA (consisting of a leader sequence, 19 nucleotides of double-stranded RNA, and two 3-prime terminal uridines). The leader sequences were removed by ribonuclease digestion, and the DNA templates were removed by deoxyribonuclease treatment. The resulting double-stranded siRNA product was purified by binding and elution from the supplied glass fiber column.

The MB1749 siRNA was tested *in vitro* by co-transfecting cultures of mouse Neuro2a cells with the MB 1749 anti-BACE1 siRNA along with a DNA plasmid (pTracerBace) that was constructed for these testing purposes. The pTracerBace plasmid contains an expression cassette for human BACE1 cDNA and an additional expression cassette for enhanced green fluorescent protein (eGFP). Neuro2a cells were plated into 6-well plates and grown to 50-70% confluence under standard culture conditions (DMEM medium with 10% fetal calf serum, at 37°C in a humidified incubator with 5% carbon dioxide). Cells were then transfected with MB 1749 siRNA using Transit-TKO transfection reagent (Mirus Catalog Number 2154) and transfected with the pTracerBace plasmid using Transit-Neuro transfection reagent (Mirus Catalog Number 2144) following the manufacturer's recommendations. Cells were fed with additional growth medium after 24 hours. After about 42 hours, total cellular RNA was harvested from the cells using the RNeasy Kit (Qiagen, Catalog Number 74104) following the manufacturer's protocol, including digestion of potential contaminating genomic DNA using DNase I.

Suppression of BACE1 mRNA was assessed using a quantitative reverse-transcription polymerase chain reaction (qRT-PCR) assay. Ten micrograms (10 ug) of total cellular RNA was reverse-transcribed to cDNA (Stratagene ProSTAR First Strand RT-PCR Kit, Catalog Number 200420) in a 50 microliter reaction. Parallel reactions omitting the reverse-transcriptase were used to verify lack of genomic DNA carry-over in the subsequent PCR reactions. Six microliters of the reaction products were mixed with TaqMan DNA polymerase PCR reaction reagents then evenly subdivided into three separate tubes, to which PCR primers for amplification of BACE1 cDNA, rodent GAPDH cDNA, or eGFP cDNA were added, respectively. The PCR reactions were run and amplification data collected using a RotorGene 3000 real-time PCR machine (Corbett Research, Mortlake NSW, Australia). A dilution series of reaction products from Neuro2a cells transfected with pTracerBace plasmid only (no siRNA) was used to establish a standard curve for each cDNA (BACE1, GAPDH, and eGFP), and the amount of cDNA for each gene in Neuro2a cells transfected with plasmid plus the MB1749 anti-BACE1 siRNA was calculated as a percentage of the respective standard.

This experiment was conducted on three separate occasions by separate laboratory personnel using separate cell cultures, with the results as follows:

**TABLE 8: Cell Culture Results**

| *Amt of BACE1 (% of standard)* | *Amt of GAPDH (% of standard)* | *Amt of eGFP (% of standard)* | *Relative amt of BACE1 (normalized to eGFP)* | *Percent reduction in BACE1 mRNA* |
|---|---|---|---|---|
| 3.957 | 148.884 | 109.256 | .036 | 96.4 % |
| 2.319 | n/a | 77.723 | .030 | 97.0 % |
| 12.281 | 13.538 | 68.102 | .018 | 82.0 % |

These results indicate that the MB 1749 siRNA sequence results in substantial suppression of BACE1 mRNA expression *in vitro* in a mouse neuronal cell line.

### EXAMPLE 3: Suppression of BACE1 mRNA in vitro using AAV-anti-Bace1.

An expression vector containing DNA coding for production of MB 1749 siRNA in transfected cells was genetically engineered. This vector was produced by inserting the following DNA sequence into the pSilencer 1.0-U6 plasmid (Ambion, Catalog Number 7207), between the ApaI and EcoRI restriction sites:
SEQ ID NO:15:

5'- GGCCGAAGACTGTGGCTACAACATTCTTCAAGAGA
GAATGTTGTAGCCACAGTCTTCTTTTTGAATT- 3'

The resulting plasmid contains an expression cassette consisting of the U6 RNA polymerase III promoter, the DNA sequence coding for a short, hairpin transcript corresponding to MB 1749, and an RNA polymerase III termination sequence consisting of a series of six thymine nucleotides. The effectiveness of this DNA sequence in causing transduced cells to internally produce MB1749 siRNA and thereby suppress the expression of BACE1 messenger RNA was tested in cultures of Neuro2a cells. Neuro2a cell cultures were co-transfected with the pSilencer-MB1749 plasmid and the pTracerBace plasmid using the Transit-Neural transfection reagent and procedures as described above. After about 42 hours, total cellular RNA was harvested from the cells and analyzed using quantitative real-time RT-PCR as described above. This analysis was conducted in triplicate, with the results as follows:

**TABLE 9: Analysis Results**

| *Amt of BACE1 (% of standard)* | *Amt of GAPDH (% of standard)* | *Amt of eGFP (% of standard)* | *Relative amt of BACE1 (normalized to eGFP)* | *Percent reduction in BACE1 mRNA* |
|---|---|---|---|---|
| 69.399 | 108.372 | 401.788 | 0.173 | 82.7 % |
| 75.772 | 99.640 | 564.212 | 0.134 | 86.6 % |
| 2.048 | 92.571 | 35.434 | 0.058 | 94.2 % |

These data indicate that the MB1749 siRNA sequence is generated from the MB1749 expression cassette within cells transfected with the pSilencer-MB1749 plasmid, and this expression results in substantial suppression of BACE1 mRNA expression *in vitro.*

The MB 1749 expression cassette (consisting of the RNA polymerase III promoter, the DNA sequence coding for a short, hairpin transcript corresponding to MB 1749, and an RNA polymerase III termination sequence) were engineered into an adeno-associated viral vector (GeneDetect, Inc., Auckland, New Zealand, Catalog Number GD1001-RV) with a chimeric AAV serotype 1 / 2. The resulting virus is called MDT1749.4. To verify that cells transduced with MDT1749.4 are induced to internally produce MB1749 siRNA and thereby suppress the expression of BACE1 messenger RNA, cultures of HEK293 cells (a standard laboratory cell line) were infected with MDT1749.4 virus, then transfected with pTracerBace plasmid one day later (day 2). Total cellular RNA was harvested from these cells approximately 48 hours later (on day 4) and analyzed using quantitative real-time RT-PCR as described above. As a negative control, other HEK293 cell cultures were transfected with pTracerBace plasmid and comparable AAV viral vectors that were identical to MDT1749.4 except that the coding sequence for MB1749 was scrambled and therefore not active as an siRNA against BACE1. This analysis was conducted using two separate lots of MDT1749.4 viral stocks and two separate lots of scrambled control viral stocks, with the results as follow's:

**TABLE 10: Analysis Results**

| *Viral vector lot number* | *Amt of BACE1 (% of standard)* | *Amt of GAPDH (% of standard)* | *Amt of eGFP (% of standard)* | *Relative amt of BACE1 (normalized to eGFP)* | *Percent reduction in BACE1 mRNA* |
|---|---|---|---|---|---|
| Lot 1301 (MDT 1749.4, anti-Bace1) | 2.557 | 5.119 | 6.033 | 0.424 | 57.6 % |
| Lot 1302 (MDT 1749.4, anti-Bace1) | 1.823 | 7.307 | 6.697 | 0.272 | 72.8 % |
| Lot 1311 (MDTCTRL.85 scrambled control) | 168.875 | 90.53 | 83.723 | 2.017 | -101.7 % |
| Lot 1312 (MDTCTRL.85 scrambled control) | 70.613 | 94.181 | 84.307 | 0.838 | 16.2 % |

These data indicate that the MB1749 siRNA sequence is generated within cells infected with the MDT1749.4 adeno-associated virus, and this expression results in substantial suppression of BACE1 mRNA expression *in vitro.* A test of the effects of the MDT1749.4 adeno-associated virus on the production of BACE1 enzyme and beta-amyloid generation and neuropathology is currently underway in a transgenic mouse model of Alzheimer's disease (Tg2576).

### EXAMPLE 4: Suppression of BACE1 protein enzyme activity in vitro using siRNA.

To verify that transfection of MB 1749 siRNA into cells in fact results in suppression of BACE1 protein expression as well as suppression of BACE1 messenger RNA production, BACE activity was measured in protein extracts from HEK293 cells. HEK293 cells were plated in 35 mm dishes, and co-transfected with pTracerBace plasmid and either pSilencer-MB1749 (containing the DNA code for producing MB1749 siRNA) or the original pSilencer plasmid as obtained from the manufacturer (Ambion, Catalog Number 7207, with no DNA for any siRNA inserted), using the Transit-Neural transfection reagent. After 48 hours, cells were harvested and protein extracted from the cell lysates was assayed for BACE enzyme activity using a Beta-Secretase Activity Kit (R&D Systems, Catalog Number FP002). Briefly, this assay involves adding a labeled peptide that is a substrate for BACE cleavage activity to the protein extracts. The peptide substrate is conjugated with a fluorescent reporter molecule (EDANS) and a fluorescence-quenching molecule (DABCYL), such that the physical proximity of the two molecules on the peptide prevents fluorescent emissions. Cleavage of the peptide substrate by BACE physically separates the EDANS and DABCYL molecules, allowing for release of the fluorescent signal.

The protein extracts obtained from the cell samples were of equivalent total protein concentration (0.13 mg/mL for cells transfected with pTracerBace and pSilencer-MB1749, and 0.11 mg/mL for cells transfected with pTracerBace and the "empty" pSilencer). Triplicate samples of these protein extracts were placed in wells of a microplate (supplied with the R&D Systems kit), at 50 microliters per well, to which 50 microliters of reaction buffer and 5 microliters of labeled peptide substrate were added, following the manufacturer's protocol. The samples were incubated at 37°C, and the fluorescence emitted from the wells was measured at 10 minute intervals using a Phenix fluorescent microplate reader. Results are illustrated in Figure 1.

It is important to note that this assay method is not specific to BACE1 enzyme activity, but also reflects BACE2 enzyme activity. Because the MB1749 siRNA is specific to BACE1 (having only 58% homology to BACE2 sequence [8 out of 19 bases mismatch]), any BACE2 protein production in cells is likely to be unaffected by MB 1749 transfection and continue to contribute to the overall BACE enzyme activity measured in this assay. Consequently, the assay is likely to be an underestimate of the amount of BACE1 protein suppression by MB 1749 in these cells.

These results indicate that production of active BACE1 protein is reduced in cells transfected with MB1749 siRNA.

In addition, several preclinical studies in animal models of Alzheimer's disease have shown that treatments that reduce beta-amyloid levels in the brain also result in improvements in cognitive performance (*see, for example,* Schenk, Nature Reviews Neuroscience, 3:824-828 (2002). These studies used an immunization approach, triggering reduction in beta-amyloid by an immune response against beta-amyloid, rather than prevention of beta-amyloid formation. Based on these preclinical results, human clinical trials of an anti-beta-amyloid vaccine have been initiated; however, these trials were halted when some patients developed brain inflammation (meningoencephalitis) in reaction to the therapy.

There is evidence from various *in vitro* and animal studies that suppression of BACE1 is feasible, and that it may be a safe and effective way to treat Alzheimer's disease. Roberds et al., in Human Molecular Genetics, 10:1317-24 (2001), have shown that mice lacking BACE1 expression (BACE1 knock-out mice) fail to produce beta-amyloid in their brains, but are phenotypically normal. Furthermore, Luo et al., in Nature Neuroscience 4:231-232 (2001), have shown that when BACE1 knock-out mice are bred with Tg2576 transgenic mice that over-express human amyloid precursor protein (APP), the offspring fail to produce beta-amyloid. Significantly, Ohno et al., in Neuron, 41:27-33 (2004), have now reported that crossing the Tg2576 mice with the BACE1 knock-outs also results in rescue of the offspring from beta-amyloid dependent pathology, including rescue from hippocampal memory deficits and from impaired regulation of neuronal excitability.

Basi et al., in Journal of Biological Chemistry, 278:31512-20 (2003), have shown that siRNA can be used to suppress BACE1 expression in a standard laboratory cell line (human embryonic kidney cells HEK293) and Kao et al., in Journal of Biological Chemistry, 279:1942-49 (2004), have shown that siRNA can be used to suppress the expression of BACE1 *in vitro* in cultures of primary cortical neurons from both wildtype and APP transgenic mice.

### EXAMPLE 5:

The following example is an exemplary method for constructing a plasmid DNA from which a single-stranded DNA that includes an artificial, self-complementary, adeno-associated viral vector (scAAV) encoding a biologically active RNA transcript can be made. In this example, the process for making the plasmid DNA includes eight steps. Once the plasmid DNA is made by these eight steps, then a large quantity of plasmid DNA (for example, micrograms, milligrams, or grams of plasmid DNA) can be made by methods known to those skilled in the art, such as by transforming bacteria with the plasmid, growing large quantities of the bacteria, then recovering the large quantity of plasmid DNA from the bacterial culture. From the large quantity of plasmid DNA, a large quantity of the artificial, self-complementary AAV can be made by two further steps. All of these steps are described below.

### Materials

A plasmid DNA, hereinafter called "pPvuABCBAPvu," from which the single-stranded DNA including the scAAV can be produced, is made using the following starting materials:
1) Phagemid plasmid pBluescript II KS+, commercially available from Stratagene, Inc. (LaJolla, CA), catalog #212207.
2) Plasmid pCMV-myc-cyto-GFP, commercially available from Invitrogen (Carlsbad, CA), catalog #V820-20.
3) Plasmid pAAV-LacZ, commercially available from Stratagene, Inc., (LaJolla, CA), catalog #240071.
4) Plasmid pSilencer1.0, commercially available from Ambion, Inc., (Austin, TX), catalog #7207.

As described herein below, restriction enzymes are used to cut these plasmids, using methods known to those skilled in the art. The restriction enzymes used are: AlwNI, PvuII, AvaII, PstI, KpnI, Bsu36I, ScaI, SfiI, NheI, BssSI, EcoRI, Bst11071, SpeI, and SspI, available from New England Biolabs, Inc., (Beverly, MA).

As described herein below, DNA modifying and DNA ligating enzymes are used to assemble DNA fragments into the plasmids, using methods known to those skilled in the art. These enzymes are T4 DNA polymerase, and T4 Ligase, available from Promega, Inc., (Madison, WI), catalog #M4211 and catalog #M1801 respectively.

In addition, the following custom DNA oligonucleotides are used to construct DNA fragments for ligation into the plasmids:
Oligo2Af (SEQ ID NO:16):
   5'- GAACAGAAACTGCTGCCTCAGGGTAC - 3' (26-mer) Oligo2Ar (SEQ ID NO:17):
   5'- CCTGAGGCAGCAGTTTCTGTTCTGCA - 3' (26-mer) Oligo3Af (SEQ ID NO:18):
   5'- TGGCCCAGGTGCAACTGCAAATGG - 3' (24-mer) Oligo3Ar (SEQ ID NO:19):
   5'- CTAGCCATTTGCAGTTGCACCTGGGCCATGG - 3' (31-mer)

In addition, the following custom DNA oligonucleotides are used as polymerase-chain reaction (PCR) primers, for verifying the results of the steps and selecting successful plasmid clones with which to proceed to later steps:
PCR2Cf (SEQ ID NO:20):
   5' - GGAGCCCCCGATTTAGAG - 3' (18-mer)
PCR2Cr (SEQ ID NO:21):
   5' - ACCCTGAGGCAGCAGTTTC - 3' (19-mer)

Also, the following custom DNA oligonucleotides are used as polymerase-chain reaction (PCR) primers, for producing a PCR product for insertion into a plasmid called pBlueAlwNI to produce a plasmid called pPvuAB, as described below:
PCR6Af (SEQ ID NO:22):
   5' - CTTTTTACGGTTCCTGGC - 3' (18-mer)
PCR6Ar (SEQ ID NO:23):
   5' - TGACCTGAGGGAGTGGC - 3' (17-mer)

### Method for constructing plasmid pPvuABCBAPvu:

*Step 1-1:* This is the first of two steps needed to remove a pre-existing AlwNI restriction site found in plasmid pBluescript II KS+. Plasmid pBluescript II KS+ is linearized with restriction enzyme AlwNI, which recognizes and cuts a double-stranded DNA sequence of nine nucleotides "...CAGNNNCTG...", where "N" stands for any deoxyribonucleotide. The overhanging 5-prime and 3-prime ends of the linearized plasmid are blunted by treating the plasmid with T4 DNA polymerase. The result is the loss of the three "NNN" nucleotides from the cut ends. The plasmid ends are then re-ligated with T4 DNA ligase. The result is a re-joining of the plasmid ends to now form the sequence "...CAGCTG... ". The resulting plasmid is called pBlueMod1.

*Step 1-2:* Because the DNA sequence of pBluescript II KS+ at the pre-existing AlwNI site is preceded in the 5-prime direction by another "CAG" sequence of nucleotides, the plasmid pBlueMod1 still contains an AlwNI restriction site, reading "...CAGCAGCTG...". In order to get rid of this AlwNI restriction site, the procedures of Step 1 are performed again, using plasmid pBlueMod1. That is, pBlueMod1 is linearized with enzyme AlwNI. The overhanging 5-prime and 3-prime ends of the linearized plasmid are blunted by treating the plasmid with T4 DNA polymerase. The result is the loss of the three middle "CAG" nucleotides from the cut ends. The plasmid ends are then re-ligated with T4 DNA ligase. The result is a re-joining of the plasmid ends to form the sequence "...CAGCTG... This sequence is preceded by TGG and followed by GTA, such that the resulting "TTGCAGCTGGTA" sequence (SEQ ID NO:24) no longer forms a recognition site for the AlwNI enzyme. The resulting plasmid, which no longer can be cut with AlwNI, is called pBlueMod2.

*Step 2:* The purpose of this step is to insert restriction sites into pBlueMod2 into which additional DNA fragments can be inserted in later steps. The restriction sites inserted are those for the enzymes AlwNI and Bsu36I. To construct the insert; Oligo2Af and Oligo2Ar (described above) are mixed together in a single test tube, heated to 65 degrees centigrade for 5 minutes, then allowed to cool to room temperature, causing two oligos of each type to anneal to form a short, double-stranded DNA molecule. The ends of the double-stranded DNA are treated with kinase to phosphorylate the ends, then the double-stranded DNA is ligated into pBlueMod2 plasmid DNA that has been linearized by cutting with PstI and KpnI restriction enzymes. The resulting plasmid is called pBlueAlwNI. Successful construction of pBlueAlwNI can be confirmed using PCR primers PCR2Cf and PCR2Cr, to amplify a PCR product from the putative pBlueAlwNI plasmid. A PCR product size of 433 basepairs will result from correct pBlueAlwNI plasmid clones.

*Step 3:* The purpose of this step is to remove recognition sites for the restriction enzymes PstI and BssSI from within the coding sequence for the green fluorescent protein in plasmid pCMV-myc-cyto-GFP. This is done by constructing a DNA insert that preserves the amino acid sequence of the green fluorescent protein, but uses a different DNA sequence, lacking any sequence matched by the PstI and BssSI recognition sequences. To construct the insert, Oligo3Af and Oligo3Ar (described above) are mixed together in a single test tube, heated to 65 degrees centigrade for 5 minutes, then allowed to cool to room temperature, causing two oligos of each type to anneal to form a short, double-stranded DNA molecule. The ends of the double-stranded DNA are treated with kinase to phosphorylate the ends, then the double-stranded DNA is ligated into pCMV-myc-cyto-GFP plasmid DNA that has been linearized by cutting with SfiI and NheI restriction enzymes. The resulting plasmid is called pCMV-GFP-Mod. Successful construction of pCMV-GFP-Mod can be confirmed in three ways: a) by cutting the plasmid with PstI and BssSI, which results in three DNA fragments of size 130, 555, and 4926 basepairs (because BssSI cuts the plasmid at three sites, but PstI no longer cuts the plasmid), b) by cutting the plasmid with PvuII, which results in two fragments of sizes 1055 and 4556, because the PvuII site at position #670 in pCMV-myc-cyto-GFP has been ablated, and c) by verifying that cells transfected with pCMV-GFP-Mod express a green fluorescent protein, visible by fluorescence microscopy.

*Step 4:* The purpose of this step is to obtain the CMV-GFP coding sequence from plasmid pCMV-GFP-Mod, and insert it into the pAAV-LacZ plasmid. This is done by cutting plasmid pCMV-GFP-Mod using restriction enzymes EcoRI and PvuII and recovering the resulting 1714 basepair fragment by gel electrophoresis and elution of the DNA fragment from the gel, using methods known to those skilled in the art. The ends of the 1714 basepair fragment are blunted by treatment with T4 DNA polymerase, then blunt-end ligated into pAAV-LacZ that has been linearized with restriction enzymes EcoRI and Bst1107I. The resulting clones are screened to identify a plasmid into which the insert has been ligated in the orientation such that the 3-prime end of the CMV-GFP and polyA expression cassette is located at the EcoRI restriction site. The resulting plasmid is called pAAV-U6-GFP-CMV. Successful construction of pAAV-U6-GFP-CMV can be confirmed by cutting the candidate plasmids with EcoRI and ScaI, which yield DNA fragments with sizes of 1896 basepairs and 3291 basepairs, as predicted. Clones with the insert in the reverse, undesired orientation, do not cut with EcoRI, resulting in a single DNA fragment of size 5187 basepairs.

*Step 5:* The purpose of step 5 is to insert a DNA sequence encoding for a short, hairpin RNA transcript that constitutes an siRNA targeting BACE1 (to suppress the expression of BACE1 enzyme in patients as a treatment for Alzheimer's disease) into the pAAV-U6-GFP-CMV plasmid. This is done by cutting a plasmid pSilencer-antiBACE1, into which the DNA encoding for the hairpin RNA targeting BACE1 is previously constructed, with the enzymes SpeI and EcoRI, and recovering the resulting 386 basepair fragment by gel electrophoresis and elution of the DNA fragment from the gel. Construction of the DNA encoding for the hairpin RNA targeting BACE1 has been disclosed in U.S. Patent Application Publication No. 2004/0220132 A1 (Kaemmerer). The fragment is ligated into the plasmid pAAV-U6-GFP-CMV (constructed in step 4) between the unique SpeI and EcoRI restriction sites found in pAAV-U6-GFP-CMV. The resulting plasmid is called pAAV-antiBACE1-GFP. It now contains, between two PvuII restriction sites the DNA sequences encoding for the following, in 5-prime to 3-prime order: PvuII site, AAV inverted terminal repeat sequence, U6 RNA pol III promoter, antiBACE1 hairpin sequence, U6 transcription termination sequence, reverse complement of a polyadenylation signal sequence, reverse complement of GFP protein code, reverse complement of CMV promoter, AAV inverted terminal repeat sequence, PvuII site. That is, it contains an expression cassette for the anti-BACE1 siRNA in the 5-prime to 3-prime direction, and an expression cassette for the reporter gene, GFP, in reverse direction.

*Step 6:* The purpose of this step is to obtain from pAAV-antiBACE1-GFP the DNA fragment extending from the 5-prime PvuII site and 5-prime AAV inverted terminal repeat sequence through the reverse complement of the CMV promoter, but not including the 3-prime AAV inverted terminal repeat. This fragment is then inserted into pBlueAlwNI between the PstI and Bsu36I restriction sites, to produce a plasmid called pPvuAB. The fragment from pAAV-antiBACE1-GFP is obtained by PCR amplification of the DNA sequence using PCR6Af and PCR6Ar as primers (described above). The resulting 2549 basepair PCR product includes PstI and Bsu36I restriction sites at its ends, due to the design of the PCR primers PCR6Af and PCR6Ar. The fragment is cut with these enzymes, then ligated into pBlueAlwNI that has been linearized with PstI and Bsu36I. Successful construction of pPvuAB can be confirmed by cutting candidate plasmids with restriction enzyme SspI, which results in three fragments of size 130, 942, and 4335 for successful clones, three fragments of size 130, 2350, and 2927 for unsuccessful clones with the insert in the undesired orientation, and two fragments of size 130 and 2780 for unsuccessful clones that do not acquire the insert.

*Step 7:* The purpose of this step is to insert a fragment from pAAV-antiBACE1-GFP into the plasmid pBlueAlwNI, this time with the fragment consisting of the entire DNA sequence between the PvuII restriction sites in pAAV-antiBACE1-GFP. This is done by cutting plasmid pAAV-antiBACE1-GFP with restriction enzyme PvuII, and recovering the 2590 basepair DNA fragment by gel electrophoresis and elution from the gel. The fragment is then blunt-end ligated into pBlueAlwNI plasmid that has been linearized using AlwNI enzyme at the unique restriction site introduced in step 2. Plasmids with the insert in the desired orientation can be identified by cutting with the restriction enzymes EcoRI and PstI, which results in two fragments of size 539 and 4975 basepairs in the plasmids with the insert in the desired orientation, but fragments of size 2059 and 3455 in plasmids with the insert in the undesired orientation and one fragment of size 2924 in plasmids that have no insert. The resulting plasmid with the insert in the desired orientation is called pPvuABC. It contains the DNA sequences encoding for the following, in 5-prime to 3-prime order: PvuII site, AAV inverted terminal repeat sequence, U6 RNA pol III promoter, antiBACE1 hairpin sequence, U6 transcription termination sequence, reverse complement of a polyadenylation signal sequence, reverse complement of GFP protein code, reverse complement of CMV promoter, AAV inverted terminal repeat sequence, AlwNI site. The 3-prime half of the original AlwNI site in pBlueAlwNI has been restored by the ligation of the insert, because the 3-prime end of the insert re-supplies the "CAG...... " portion of the AlwNI recognition pattern.

*Step 8:* The final step in the preparation of plasmid pPvuABCBAPvu is to blunt clone the PvuAB portion from plasmid pPvuAB into plasmid pPvuABC producing the desired plasmid pPvuABCBAPvu. This is accomplished by cutting plasmid pPvuAB with the enzymes PstI and Bsu36I, and recovering the resulting fragment of size 2497 basepairs. The ends of this insert are blunted by treatment with T4 DNA polymerase. The insert is then blunt-end ligated into plasmid pPvuABC that has been linearized by cutting with restriction enzyme AlwNI. The resulting plasmids are screened to identify plasmids with the desired orientation of the insert by cutting with PvuII. When cut with PvuII, plasmid clones with the desired insert yield five DNA fragments of sizes 182, 237, 586, 1921, and 5085 basepairs. Plasmid clones with the insert in the undesired orientation yield five DNA fragments of sizes 182, 586, 1921, 2595, and 2727 basepairs in length. Plasmid clones with no insert yield five fragments of sizes 182, 232, 586, 1921, and 2590 basepairs in length. The resulting plasmid with the insert in the desired orientation contains the 5085 basepair DNA sequence encoding for the following, in 5-prime to 3-prime order: PvuII site, AAV inverted terminal repeat sequence, U6 RNA pol III promoter, antiBACE1 hairpin sequence, U6 transcription termination sequence, reverse complement of a polyadenylation signal sequence, reverse complement of GFP protein code, reverse complement of CMV promoter, AAV inverted terminal repeat sequence, CMV promoter, GFP protein code, polyadenylation signal sequence, reverse complement of U6 transcription termination sequence, reverse complement of antiBACE1 hairpin sequence, reverse complement of U6 RNA pol III promoter, AAV inverted terminal repeat sequence, PvuII site.

### Method for constructing artificial AAV from plasmid pPvuABCBAPvu:

To obtain single-stranded DNA that includes an artificial, self-complementary, adeno-associated viral vector (scAAV) encoding an anti-BACE1 RNA transcript from plasmid pPvuABCBAPvu, only two simple steps are required.

*Step 1:* The plasmid pPvuABCBAPvu is cut with restriction enzyme PvuII, and the DNA fragment of size 5085 basepairs is recovered and purified by methods known to those skilled in the art, such as gel electrophoresis and elution from the gel.

*Step 2:* The 5085 double-stranded DNA fragment is put into dilute, aqueous solution, warmed to 99 degrees centigrade for 5 to 10 minutes, then allowed to cool to room temperature. The heating causes the two strands of the double-stranded DNA to separate, or "melt" apart. Once separated and in a dilute solution, these two strands each are more likely to self-anneal, forming a large DNA hairpin of about 2542 basepairs in length, rather than to reanneal with their complementary strand forming a double helix of size 5085 basepairs. The resulting large DNA hairpin molecules, from each strand of the original double stranded DNA, are identical, and each is the desired single-stranded, self-complementary, artificial AAV vector.

In one embodiment of the present invention, the resulting single-stranded DNA is then packaged into pegylated immunoliposomes for intravenous or intra-arterial administration to an animal or human patient to cause the single-stranded DNA to be transported across the blood-brain barrier and into cells within the central nervous system of the animal or human patient to provide a treatment for a disease of the central nervous system. For example, the resulting single-stranded DNA produced from a pPvuABCBAPvu plasmid containing a DNA sequence encoding for an RNA hairpin that cells process into a small, interfering RNA targeting BACE1 enzyme can be delivered across the blood-brain barrier in human patients as a treatment for Alzheimer's disease.

In a comparable manner, treatments for diseases caused by an absence or deficiency in a gene product (such as inborn errors of metabolism, including lysosomal storage diseases) can be made and delivered to animal or human patients, as embodiments of the present invention, by inserting the coding sequence for the missing or deficient gene product operably linked to a promoter sequence at the 5-prime end and a polyadenylation signal sequence at the 3-prime end, into plasmids pPvuAB and pPvuABC, in the place of the coding sequences for the siRNA and the green fluorescent protein. From these two plasmids, a single plasmid of the form pPvuABCBAPvu may then be made using the method described in Step 8 above. Then self-complementary AAV can be produced from that pPvuABCBAPvu plasmid using the method described above, and packaged into pegylated immunoliposomes for intravenous or intra-arterial administration to an animal or human patient to cause the single-stranded DNA to be transported across the blood-brain barrier and into cells within the central nervous system. This administration thereby provides a treatment for the central nervous system manifestations of the disease caused by the gene deficiency, as well as the systemic manifestations of the disease.

### EXAMPLE 6

An artificial adeno-associated virus vector as described in Example 5 is prepared and packaged in a liposome in a manner similar to that described in U.S. Patent No. 6,372,250 (Pardridge) to provide a composition. The composition is injected into the tail veins of TG2576 transgenic mice, which are an accepted animal model for human Alzheimer's disease. The mice are followed up to an age at which substantial beta-amyloid plaque is expected to be observed in the undosed controls. The mice are then sacrificed and less beta-amyloid plaque is observed for the dosed mice than for the undosed mice.

### EXAMPLE 7

The following example is an exemplary method for preparing a double-stranded artificial AAV vector according to one embodiment of the present invention. In this example, the plasmid pAAV-antiBACE1-GFP was constructed by following the methods of step 1 through step 5 of EXAMPLE 5, as disclosed herein. The resulting plasmid contains a desired DNA segment between two PvuII restriction sites. Specifically, the plasmid contains a DNA sequence, in 5-prime to 3-prime order, encoding for a PvuII site, an AAV inverted terminal repeat sequence, a U6 RNA pol III promoter, an antiBACE1 hairpin sequence, a U6 transcription termination sequence, a reverse complement of a polyadenylation signal sequence, a reverse complement of GFP protein code, a reverse complement of CMV promoter, an AAV inverted terminal repeat sequence, and a PvuII site. That is, it contains an expression cassette for the anti-BACE1 siRNA in the 5-prime to 3-prime direction, and an expression cassette for the reporter gene, GFP, in reverse direction (see Figure 5).

To isolate the expression cassette flanked by the AAV ITRs in plasmid pAAV-antiBACE1-GFP, 75 µg of the plasmid was digested with the restriction enzymes PvuII and ScaI using methods well known to those skilled in the art. ScaI digests the plasmid backbone into two smaller fragments allowing further discrimination between the plasmid backbone and the desired insert. The linear fragment was gel purified using the Qiaex II gel purification kit from Qiagen. The percent recovery and quality of the linear fragment was determined using spectrophotometry by measuring the absorbance at 260 and 280 nm. Through this quantification, it was determined that 60% of the starting product was recovered and the purified linear fragment was of sufficient quality (A₂₆₀/A₂₈₀ = 1.9).

To allow the AAV ITRs to assume a secondary structure, 4.5 µg of the linear fragment (90 ng/µl) was thermally treated by heating to 65°C for 10 minutes and allowed to cool slowly to room temperature for a minimum of 10 minutes. As a control for use in the subsequent *in vitro* experiment, untreated linear fragment was allowed to sit at room temperature for the 10 minutes that the heated fragment was cooling. The expected conformation of the untreated linear fragment and the treated (heated and cooled) fragment are shown in Figure 3 and Figure 4, respectively. Immediately following incubation at room temperature, these artificial AAV vectors were transiently transfected into HEK293T cells.

The purpose of the transfection experiment was to compare the longevity of EGFP expression in cells transfected with the artificial AAV vectors to the longevity of EGFP expression in cells transfected with a circular plasmid containing an EGFP expression cassette (pTRACER-CMV2, available from Invitrogen Corporation, Carlsbad, CA). This experiment was designed to observe whether expression of EGFP in cells treated with the artificial AAV vectors of the present invention could persist longer than expression of EGFP in cells treated with the circular plasmid pTRACER-CMV2.

### METHOD: Transfection of HEK293 cells:

HEK293T (ATCC #CRL 11268) were cultured in DMEM containing 4.5 g/L glucose and supplemented with 10% FBS and penicillin and streptomycin. The day prior to transfection HEK293T cells were seeded into 6-well tissue culture plates at a density of 5x10⁵ cells/well. This seeding density yielded wells that were approx. 80% confluent the day of transfection.

HEK293T cells were transfected with Transit TKO (Mirus) following the manufacturers recommended protocol. Briefly, 6 µL of Transit-TKO was added to 200 µL of Opti-Mem reduced serum media in a 5 mL polystyrene tube, followed by vortex mixing. The diluted transfection reagent was incubated at room temperature for 15 minutes. One-microgram of the appropriate DNA was added to each tube, the DNA was gently mixed and incubated at room temperature for 20 minutes. Samples included: (1) mock transfected (H₂O); (2) pTRACER plasmid (1µg); (3) artificial AAV vector (1µg); and (4) thermally treated (e.g., heated and cooled) artificial AAV vector (1µg). Following incubation, the media on the cells was removed and replaced with 2 mL of normal growth media (DMEM, high glucose, 10% FBS, penicillin/streptomycin). The DNA complexes were slowly added dropwise to the cells and mixed by gentle rocking. The transfected cells were incubated at 37°C in a humidified incubator containing 5% CO₂. To follow EGFP expression, photographs of the transfected cells were taken every 2-4 days up to 30 days post-transfection. An area that was representative of the entire well was photographed. On the day of photographing the media was replaced with fresh normal growth media on all of the transfected wells.

To allow for comparison of EGFP expression in the transfected cells, all of the digital photographs were taken using the same parameters. Analysis of the images revealed a persistence of EGFP expression from the artificial AAV vectors. In addition, the heated and cooled artificial AAV vector was substantially more effective at transfecting the cells and producing persistence of EGFP expression. This is evident in the images collected at 6 and 23 days post-transfection shown in Figure 6.

As can be seen in Figure 6, greater than 95% plasmid-derived EGFP expression was lost by 12 days post-transfection. The artificial AAV vector produced without the final thermal treatment step (e.g., heating and cooling) yielded EGFP expression in only a few cells; however, the EGFP expression persisted in those cells. The observed persistence can be interpreted as an indication of formation of the secondary structure of the AAV-ITRs in a minority of transfected cells, followed by persistent expression of EGFP due to the secondary structure achieved the ITRs in the cells. The artificial AAV vector produced according to the disclosed method including the thermal treatment step (e.g., heating and cooling) resulted in continued EGFP expression at 27 days post-transfection. The continued expression can be interpreted as indicative of formation of the secondary structures of the AAV-ITRs by the thermal treatment step (e.g., heating and cooling) such that these structures, conducive to persistent expression of EGFP, were present in substantially all cells transduced by the artificial AAV vector.

The experiment was terminated 27 days post-transfection because significant cell loss began occurring after this time point. Comparing expression from the thermally-treated artificial AAV vector to that from the plasmid, a greater than 100% increase (in number of days) in the persistence of expression was obtained from the artificial AAV vector.

This example illustrates that significant and stable expression can be obtained from an artificial AAV vector that is an embodiment of the subject invention, produced according to the disclosed methods. Expression from this artificial AAV vector was observed to persist much longer than expression from a circular plasmid.

The foregoing detailed description and examples have been given for clarity of understanding only. No unnecessary limitations are to be understood therefrom. The invention is not limited to the exact details shown and described, for variations obvious to one skilled in the art will be included within the invention defined by the claims.

## Claims

1. A medical system for delivering single stranded DNA across a blood-brain barrier, the system comprising:
a neurovascular catheter having a distal end to be positioned in a blood vessel supplying a patient's brain;
a composition comprising:
an artificial adeno-associated virus (AAV) vector comprising single stranded DNA encoding a biologically active agent; and
a component to deliver at least the single stranded DNA across the blood-brain barrier, wherein:
a) said single stranded DNA encoding the biologically active agent is SEQ ID NO: 2; or
b) said AAV is selected from SEQ ID NOs: 8-11; and
a means for delivering said composition to the catheter.

2. The medical system of claim 1 further comprising an implantable pump for delivery of the composition to the patient's blood stream.

3. The medical system of claim 1 or claim 2 wherein the artificial AAV vector comprises AAV-ITRs at the 5-prime and 3-prime ends.

4. The medical system of any one of claims 1 to 3 wherein the artificial AAV vector comprises, in 5-prime to 3-prime order:
a 5-prime AAV-ITR;
the single stranded DNA;
an internal AAV-ITR;
a reverse complement of the single stranded DNA; and
a 3-prime AAV-ITR.

5. The medical system of claim 4 wherein the artificial AAV vector has been thermally treated in at least one heating and cooling cycle.

6. The medical system of claim 1 wherein the component to deliver at least the single stranded DNA across the blood-brain barrier comprises:
a receptor-specific liposome having an exterior surface and an internal compartment;
wherein the artificial adeno-associated virus (AAV) vector is located within the internal compartment of the liposome;
one or more blood-brain barrier and brain cell membrane targeting agents; and
one or more conjugation agents wherein each targeting agent is connected to the exterior surface of the liposome via at least one of the conjugation agents.

7. The medical system of claim 6 further comprising an implantable pump for delivery of the composition to the patient's blood stream.

8. The medical system of claim 6 or claim 7 wherein the exterior surface of the liposome defines a sphere having a diameter of at most 200 nanometers.

9. The medical system of claim 6 wherein at least 5 and at most 1000 blood-brain barrier or brain cell membrane targeting agents are conjugated to the surface of the liposome.

10. The medical system of claim 6 wherein at least 25 and at most 40 blood-brain barrier or brain cell membrane targeting agents are conjugated to the surface of the liposome.

11. The medical system of claim 6 wherein the conjugation agent is selected from the group consisting of polyethylene glycol, sphingomyelin, biotin, streptavidin, organic polymers, and combinations thereof.

12. The medical system of claim 6 wherein the molecular weight of the conjugation agent is at least 1000 Daltons and at most 50,000 Daltons.

13. The medical system of claim 6 wherein the single stranded DNA encodes a short hairpin RNA.

14. A composition for delivering single-stranded DNA across a blood-brain barrier for expression in the brain, the composition comprising:
a) a receptor-specific liposome, wherein the receptor-specific liposome comprises:
a liposome having an exterior surface and an internal compartment;
an artificial adeno-associated virus (AAV) vector located within the internal compartment of the liposome, wherein the artificial AAV vector comprises single stranded DNA encoding a biologically active agent;
one or more blood-brain barrier and brain cell membrane targeting agents; and
one or more conjugation agents wherein each targeting agent is connected to the exterior surface of the liposome via at least one of the conjugation agents; or
b) a receptor-specific nanocontainer, wherein the receptor-specific nanocontainer comprises:
a nanocontainer having an exterior surface and an internal compartment;
an artificial adeno-associated virus (AAV) vector located within the internal compartment of the nanocontainer, wherein the artificial AAV vector comprises single stranded DNA encoding a biologically active agent;
one or more receptor specific targeting agents that target the receptor located on the cell; and
one or more conjugation agents wherein each targeting agent is connected to the exterior surface of the nanocontainer via at least one of the conjugation agents; or
c) an artificial adeno-associated virus (AAV) vector comprising single stranded DNA encoding a biologically active agent; and
a component to deliver at least the single-stranded DNA across the blood-brain barrier,
wherein:
said single stranded DNA encoding the biologically active agent is SEQ ID NO: 2; or
said AAV is selected from SEQ ID NOs: 8-11.

15. The composition of claim 14 wherein the exterior surface of the liposome defines a sphere having a diameter of at most 200 nanometers.

16. The composition of claim 14 wherein at least 5 and at most 1000 blood-brain barrier or brain cell membrane targeting agents are conjugated to the surface of the liposome.

17. The composition of claim 14 wherein at least 25 and at most 40 blood-brain barrier or brain cell membrane targeting agents are conjugated to the surface of the liposome.

18. The composition of claim 14 wherein the conjugation agent is selected from the group consisting of polyethylene glycol, sphingomyelin, biotin, streptavidin, organic polymers, and combinations thereof.

19. The composition of claim 14 wherein the molecular weight of the conjugation agent is at least 1000 Daltons and at most 50,000 Daltons.

20. The composition of claim 14 wherein the exterior surface of the nanocontainer defines a sphere having a diameter of at most 200 nanometers.

21. The composition of any one of claims 14 to 20 wherein the artificial AAV vector comprises AAV-ITRs at the 5-prime and 3-prime ends.

22. The composition of any one of claims 14 to 21 wherein the artificial AAV vector comprises, in 5-prime to 3-prime order:
a 5-prime AAV-ITR;
the single stranded DNA;
an internal AAV-ITR;
a reverse complement of the single stranded DNA; and
a 3-prime AAV-ITR.

23. The composition of claim 14 wherein the artificial AAV vector has been thermally treated in at least one heating and cooling cycle.

24. The composition of claim 14 for delivering single stranded DNA across a blood-brain barrier for expression in the brain, the composition comprising:
an artificial adeno-associated virus (AAV) vector comprising single stranded DNA encoding a biologically active agent; and
a component to deliver at least the single stranded DNA across the blood-brain barrier, wherein:
a) the biologically active agent is encoded by SEQ ID NO: 2.

25. The composition of claim 24 wherein the artificial AAV vector comprises a sequence selected from the group consisting of SEQ ID NOs: 8-11.

26. The composition of claim 14 wherein the single stranded DNA encoding the biologically active agent comprises a sequence encoded by SEQ ID NO: 2.

27. The composition of claim 24 wherein the single stranded DNA encodes a short hairpin RNA.

28. An artificial adeno-associated virus (AAV) vector comprising, in 5-prime to 3-prime order:
a 5-prime AAV-ITR;
a single stranded DNA encoding a biologically active agent;
an internal AAV-ITR;
a reverse complement of the DNA encoding the biologically active agent; and
a 3-prime AAV-ITR, wherein:
a) said single stranded DNA encoding the biologically active agent is encoded by SEQ ID NO: 2; or
b) said artificial AAV is selected from SEQ ID NOs: 8-11.

29. The vector of claim 28 wherein the artificial AAV vector comprises a sequence selected from the group consisting of SEQ ID NOs: 10-11.

30. The vector of claim 28 wherein the single stranded DNA encoding the biologically active agent comprises a sequence of SEQ ID NO: 2.

31. The vector of claim 28 wherein the single stranded DNA encodes a short hairpin RNA.

32. The artificial adeno-associated virus (AAV) vector of claim 28 wherein:
a) said DNA encoding the biologically active agent is encoded by SEQ ID NO: 2; or
b) said artificial AAV is selected from SEQ ID NOs: 10-11.

33. The vector of claim 32 wherein the artificial AAV vector has been thermally treated in at least one heating and cooling cycle.

34. The vector of claim 32 wherein the DNA encodes a short hairpin RNA.

35. The composition of any one of claims 14 to 27 for use in the treatment of Alzheimer's disease.

## Patentansprüche

1. Medizinisches System zum Verabreichen einer einzelsträngigen DNA über eine Blut-Gehirn-Schranke hinweg, wobei das System enthält:
einen neurovaskulären Katheter mit einem distalen Ende, der in einem Blutgefäß positioniert werden soll, das das Gehirn des Patienten versorgt;
eine Zusammensetzung, die enthält:
einen künstlichen adeno-assoziierten-Virus-(AAV)-Vektor, der eine einzelsträngige DNA enthält, die ein biologisch aktives Mittel codiert; und
eine Komponente, um mindestens die einzelsträngige DNA über die Blut-Gehirn-Schranke hinweg zu verabreichen, wobei:
a) die einzelsträngige DNA, die ein biologisch aktives Mittel codiert, SEQ ID Nr: 2 ist; oder
b) der AAV ausgewählt ist aus SEQ ID Nr: 8-11; und Mittel zum Verabreichen der Zusammensetzung in den Katheter.

2. Medizinisches System nach Anspruch 1, das ferner eine implantierbare Pumpe zur Verabreichung der Zusammensetzung in den Blutstrom des Patienten enthält.

3. Medizinisches System nach Anspruch 1 oder 2, wobei der künstliche AAV-Vektor AAV-ITRs an den 5- und 3-Enden enthält.

4. Medizinisches System nach einem der Ansprüche 1 bis 3, wobei der künstliche AAV-Vektor in der Reihenfolge 5-Ende bis 3-Ende enthält:
eine 5-Ende-AAV-ITR;
die einzelsträngige DNA;
eine interne AAV-ITR;
ein Umkehrkomplement der einzelsträngigen DNA; und
eine 3-Ende-AAV-ITR.

5. Medizinisches System nach Anspruch 4, wobei der künstliche AAV-Vektor in mindestens einem Erwärmungs- und Abkühlungszyklus thermisch behandelt worden ist.

6. Medizinisches System nach Anspruch 1, wobei die Komponente, um mindestens die einzelsträngige DNA über die Blut-Gehirn-Schranke hinweg zu verabreichen, enthält:
ein rezeptorspezifisches Liposom mit einer äußeren Oberfläche und einem inneren Kompartiment;
wobei der künstliche adeno-assoziierte-Virus-Vektor (AAV-Vektor) innerhalb des inneren Kompartiments des Liposoms angeordnet ist;
ein oder mehrere zielmittel für die Blut-Gehirn-Schranke und die Gehirnzellmembran; und
ein oder mehrere Konjugationsmittel, wobei jedes zielmittel mit der äußeren Oberfläche des Liposoms über mindestens eines der Konjugationsmittel verbunden ist.

7. Medizinisches System nach Anspruch 6, das ferner eine implantierbare Pumpe zur Verabreichung der Zusammensetzung in den Blutstrom des Patienten enthält.

8. Medizinisches System nach Anspruch 6 oder 7, wobei die äußere Oberfläche des Liposoms eine Kugel mit einem Durchmesser von höchstens 200 Nanometer definiert.

9. Medizinisches System nach Anspruch 6, wobei mindestens 5 und höchstens 1000 zielmittel für die Blut-Gehirn-Schranke oder für die Gehirnzellmembran an der Oberfläche des Liposoms konjugiert sind.

10. Medizinisches System nach Anspruch 6, wobei mindestens 25 und höchstens 40 Zielmittel für die Blut-Gehirn-Schranke oder für die Gehirnzellmembran an der Oberfläche des Liposoms konjugiert sind.

11. Medizinisches System nach Anspruch 6, wobei das Konjugationsmittel ausgewählt ist aus der Gruppe bestehend aus Polyethylenglycol, Sphingomyelin, Biotin, Streptavidin, organischen Polymeren und Kombinationen hiervon.

12. Medizinisches System nach Anspruch 6, wobei das Molekulargewicht des Konjugationsmittels mindestens 1000 Dalton und höchstens 50000 Dalton beträgt.

13. Medizinisches System nach Anspruch 6, wobei die einzelsträngige DNA eine kurze Haarnadel-RNA codiert.

14. Zusammensetzung zum Verabreichen einer einzelsträngigen DNA über eine Blut-Gehirn-Schranke hinweg zur Expression in dem Gehirn, wobei die Zusammensetzung enthält:
a) ein rezeptorspezifisches Liposom, wobei das rezeptorspezifische Liposom enthält:
ein Liposom mit einer äußeren Oberfläche und einem inneren Kompartiment;
einen künstlichen adeno-assoziierten-Virus-Vektor (AAV-Vektor), der innerhalb des inneren Kompartiments des Liposoms angeordnet ist, wobei der künstliche AAV-Vektor eine einzelsträngige DNA enthält, die ein biologisch aktives Mittel codiert;
ein oder mehrere Zielmittel für die Blut-Gehirn-Schranke und die Gehirnzellmembran; und
ein oder mehrere Konjugationsmittel, wobei jedes Zielmittel mit der äußeren Oberfläche des Liposoms über mindestens eines der Konjugationsmittel verbunden ist; oder
b) einen rezeptorspezifischen Nanobehälter, wobei der rezeptorspezifische Nanobehälter enthält:
einen Nanobehälter mit einer äußeren Oberfläche und einem inneren Kompartiment;
einen künstlichen adeno-assoziierten-Virus-Vektor(AAV-Vektor), der innerhalb des inneren Kompartiments des Nanobehälters angeordnet ist, wobei der künstliche AAV-Vektor eine einzelsträngige DNA enthält, die ein biologisch aktives Mittel codiert;
ein oder mehrere rezeptorspezifische zielmittel, die den Rezeptor ansteuern, der auf der Zelle angeordnet ist; und
ein oder mehrere Konjugationsmittel, wobei jedes zielmittel mit der äußeren Oberfläche des Nanobehälters über mindestens eines der Konjugationsmittel verbunden ist; oder
c) einen künstlichen adeno-assoziierten-Virus-Vektor (AAV-Vektor), der eine einzelsträngige DNA enthält, die ein biologisch aktives Mittel codiert; und
eine Komponente, um mindestens die einzelsträngige DNA über die Blut-Gehirn-Schranke hinweg zu verabreichen, wobei:
die einzelsträngige DNA, die ein biologisch aktives Mittel codiert, SEQ ID Nr: 2 ist; oder
der AAV ausgewählt ist aus SEQ ID Nr: 8-11;

15. Zusammensetzung nach Anspruch 14, wobei die äußere Oberfläche des Liposoms eine Kugel mit einem Durchmesser von höchstens 200 Nanometer definiert.

16. Zusammensetzung nach Anspruch 14, wobei mindestens 5 und höchstens 1000 Zielmittel für die Blut-Gehirn-Schranke oder für die Gehirnzellmembran an der Oberfläche des Liposoms konjugiert sind.

17. Zusammensetzung nach Anspruch 14, wobei mindestens 25 und höchstens 40 Zielmittel für die Blut-Gehirn-Schranke oder für die Gehirnzellmembran an der Oberfläche des Liposoms konjugiert sind.

18. Zusammensetzung nach Anspruch 14, wobei das Konjugationsmittel ausgewählt ist aus der Gruppe bestehend aus Polyethylenglycol, Sphingomyelin, Biotin, Streptavidin, organischen Polymeren und Kombinationen derselben.

19. Zusammensetzung nach Anspruch 14, wobei das Molekulargewicht des Konjugationsmittels mindestens 1000 Dalton und höchstens 50000 Dalton beträgt.

20. Zusammensetzung nach Anspruch 14, wobei die äußere Oberfläche des Nanobehälters eine Kugel mit einem Durchmesser von höchstens 200 Nanometer definiert.

21. Zusammensetzung nach einem der Ansprüche 14 bis 20, wobei der künstliche AAV-Vektor AAV-ITRs an den 5- und 3-Enden enthält.

22. Zusammensetzung nach einem der Ansprüche 14 bis 21, wobei der künstliche AAV-Vektor in der Reihenfolge 5-Ende bis 3-Ende enthält:
eine 5-Ende AAV-ITR;
die einzelsträngige DNA;
eine interne AAV-ITR;
ein Umkehrkomplement der einzelsträngigen DNA; und
eine 3-Ende AAV-ITR.

23. Zusammensetzung nach Anspruch 14, wobei der künstliche AAV-Vektor in mindestens einem Erwärmungs- und Abkühlungszyklus thermisch behandelt worden ist.

24. Zusammensetzung nach Anspruch 14 zum Verabreichen einer einzelsträngigen DNA über eine Blut-Gehirn-Schranke hinweg zur Expression in dem Gehirn, wobei die Zusammensetzung enthält:
einen künstlichen adeno-assoziierten-Virus-Vektor (AAV-Vektor), der eine einzelsträngige DNA enthält, die ein biologisch aktives Mittel codiert; und
eine Komponente, um mindestens die einzelsträngige DNA über die Blut-Gehirn-Schranke hinweg zu verabreichen, wobei:
a) das biologisch aktive Mittel durch SEQ ID Nr: 2 codiert ist.

25. Zusammensetzung nach Anspruch 24, wobei der künstliche AAV-Vektor eine Sequenz enthält, die ausgewählt ist aus der Gruppe bestehend aus SEQ ID Nr: 8-11.

26. Zusammensetzung nach Anspruch 14, wobei die einzelsträngige DNA, die das biologisch aktive Mittel codiert, eine Sequenz enthält, die durch SEQ ID Nr: 2 codiert ist.

27. Zusammensetzung nach Anspruch 24, wobei die einzelsträngige DNA eine kurze Haarnadel-RNA codiert.

28. Künstlicher adeno-assoziierter-Virus-Vektor (AVV-Vektor), der in der Reihenfolge 5-Ende bis 3-Ende enthält:
eine 5-Ende AAV-ITR;
eine einzelsträngige DNA, die das biologisch aktive Mittel codiert;
eine interne AAV-ITR;
ein Umkehrkomplement der DNA, die das biologisch aktive Mittel codiert; und
eine 3-Ende AAV-ITR, wobei:
a) die einzelsträngige DNA, die das biologisch aktive Mittel codiert, durch SEQ ID Nr: 2 codiert ist; oder
b) der künstliche AAV ausgewählt ist aus SEQ ID Nr: 8-11.

29. Vektor nach Anspruch 28, wobei der künstliche AAV eine Sequenz enthält, die ausgewählt ist aus der Gruppe bestehend aus SEQ ID Nr: 10-11.

30. Vektor nach Anspruch 28, wobei die einzelsträngige DNA, die das biologisch aktive Mittel codiert, eine Sequenz von SEQ ID Nr: 2 enthält.

31. Vektor nach Anspruch 28, wobei die einzelsträngige DNA eine kurze Haarnadel-RNA codiert.

32. Künstlicher adeno-assoziierter-Virus-Vektor (AVV-Vektor) nach Anspruch 28, wobei:
a) die DNA, die das biologisch aktive Mittel codiert, durch SEQ ID Nr: 2 codiert ist; oder
b) der künstliche AAV ausgewählt ist aus SEQ ID Nr: 10-11.

33. Vektor nach Anspruch 32, wobei der künstliche AAV-Vektor in mindestens einem Erwärmungs- und Abkühlungszyklus thermisch behandelt worden ist.

34. Vektor nach Anspruch 32, wobei die DNA eine kurze Haarnadel-RNa codiert.

35. Zusammensetzung nach einem der Ansprüche 14 bis 27 für die Verwendung in der Behandlung der Alzheimerkrankheit.

## Revendications

1. Système médical destiné à administrer un ADN simple brin à travers une barrière sang-cerveau, le système comportant :
un cathéter neurovasculaire ayant une extrémité distale à positionner dans un vaisseau sanguin alimentant le cerveau d'un patient ;
une composition comportant :
un vecteur de virus adéno-associé artificiel (AAV) comportant l'ADN simple brin codant un agent biologiquement actif ; et
un composant pour administrer au moins l'ADN simple brin à travers la barrière sang-cerveau, dans lequel :
a) ledit ADN simple brin codant l'agent biologiquement actif est la SEQ ID N° : 2 ; ou
b) ledit AAV est choisi parmi les SEQ ID N° : 8 à 11 ; et des moyens pour administrer ladite composition au cathéter.

2. Système médical selon la revendication 1, comportant en outre une pompe implantable pour l'administration de la composition au courant sanguin du patient.

3. Système médical selon la revendication 1 ou la revendication 2, dans lequel le vecteur AAV artificiel comporte des AAV-ITR aux extrémités 5-prime et 3-prime.

4. Système médical selon l'une quelconque des revendications 1 à 3, dans lequel le vecteur AAV artificiel comporte, dans le sens 5-prime à 3-prime :
une AAV-ITR 5-prime ;
l'ADN simple brin ;
une AAV-ITR interne ;
un complément inverse de l'ADN simple brin ; et
une AAV-ITR 3-prime.

5. Système médical selon la revendication 4, dans lequel le vecteur AAV artificiel a été traité thermiquement pendant au moins un cycle de chauffage et de refroidissement.

6. Système médical selon la revendication 1, dans lequel le composant pour administrer au moins l'ADN simple brin à travers la barrière sang-cerveau comporte :
un liposome spécifique à un récepteur ayant une surface extérieure et un compartiment interne ;
dans lequel le vecteur de virus adéno-associé artificiel (AAV) est situé à l'intérieur du compartiment interne du liposome ;
un ou plusieurs agents de ciblage de barrière sang-cerveau et de membrane cellulaire du cerveau ; et
un ou plusieurs agents de conjugaison, chaque agent de ciblage étant relié à la surface extérieure du liposome via au moins un des agents de conjugaison.

7. Système médical selon la revendication 6, comportant en outre une pompe implantable pour l'administration de la composition au courant sanguin du patient.

8. Système médical selon la revendication 6 ou la revendication 7, dans lequel la surface extérieure du liposome définit une sphère ayant un diamètre d'au plus 200 nanomètres.

9. Système médical selon la revendication 6, dans lequel au moins 5 et au plus 1 000 agents de ciblage de barrière sang-cerveau ou de membrane cellulaire du cerveau sont conjugués à la surface du liposome.

10. Système médical selon la revendication 6, dans lequel au moins 25 et au plus 40 agents de ciblage de barrière sang-cerveau ou de membrane cellulaire du cerveau sont conjugués à la surface du liposome.

11. Système médical selon la revendication 6, dans lequel l'agent de conjugaison est choisi parmi le groupe constitué de polyéthylène glycol, de sphingomyéline, de biotine, de streptavidine, de polymères organiques et de combinaisons de ceux-ci.

12. Système médical selon la revendication 6, dans lequel la masse moléculaire de l'agent de conjugaison est au moins de 1 000 Daltons et au plus de 50 000 Daltons.

13. Système médical selon la revendication 6, dans lequel l'ADN simple brin code un petit ARN en épingle à cheveux.

14. Composition destinée à administrer un ADN simple brin à travers une barrière sang-cerveau en vue d'une expression dans le cerveau, la composition comportant :
a) un liposome spécifique à'un récepteur, dans lequel le liposome spécifique à un récepteur comporte :
un liposome ayant une surface extérieure et un compartiment interne ;
un vecteur de virus adéno-associé artificiel (AAV) situé à l'intérieur du compartiment interne du liposome, dans lequel le vecteur AAV artificiel comporte l'ADN simple brin codant un agent biologiquement actif ;
un ou plusieurs agents de ciblage de barrière sang-cerveau et de membrane cellulaire du cerveau ; et
un ou plusieurs agents de conjugaison, chaque agent de ciblage étant relié à la surface extérieure du liposome via au moins un des agents de conjugaison ; ou
b) un nanoconteneur spécifique à un récepteur, ledit nanoconteneur spécifique à un récepteur comportant :
un nanoconteneur ayant une surface extérieure et un compartiment interne ;
un vecteur de virus adéno-associé artificiel (AAV) situé à l'intérieur du compartiment interné du nanoconteneur, le vecteur AAV artificiel comportant l'ADN simple brin codant un agent biologiquement actif ;
un ou plusieurs agents de ciblage spécifiques à un récepteur qui ciblent le récepteur situé sur la cellule ; et
un ou plusieurs agents de conjugaison, chaque agent de ciblage étant relié à la surface extérieure d'un nanoconteneur via au moins un des agents de conjugaison ; ou
c) un vecteur de virus adéno-associé artificiel (AAV) comportant l'ADN simple brin codant un agent biologiquement actif ; et
un composant pour administrer au moins l'ADN simple brin à travers la barrière sang-cerveau,
dans lequel :
ledit ADN simple brin codant l'agent biologiquement actif est la SEQ ID N° : 2^{h} ; ou
ledit AAV est choisi parmi les SEQ ID N° : 8 à 11.

15. Composition selon la revendication 14, dans laquelle la surface extérieure du liposome définit une sphère ayant un diamètre d'au plus 200 nanomètres.

16. Composition selon la revendication 14, dans laquelle au moins 5 et au plus 1 000 agents de ciblage de barrière sang-cerveau ou de membrane cellulaire du cerveau sont conjugués à la surface du liposome.

17. Composition selon la revendication 14, dans laquelle au moins 25 et au plus 40 agents de ciblage de barrière sang-cerveau ou de membrane cellulaire du cerveau sont conjugués à la surface du liposome.

18. Composition selon la revendication 14, dans laquelle l'agent de conjugaison est choisi parmi le groupe constitué de polyéthylène glycol, de sphingomyéline, de biotine, de streptavidine, de polymères organiques et de combinaisons de ceux-ci.

19. Composition selon la revendication 14, dans laquelle la masse moléculaire de l'agent de conjugaison est au moins de 1 000 Daltons et au plus de 50 000 Daltons.

20. Composition selon la revendication 14, dans laquelle la surface extérieure du nanoconteneur définit une sphère ayant un diamètre d'au plus 200 nanomètres.

21. Composition selon l'une quelconque des revendications 14 à 20, dans laquelle le vecteur AAV artificiel comporte des AAV-ITR aux extrémités 5-prime et 3-prime.

22. Composition selon l'une quelconque des revendications 14 à 21, dans laquelle le vecteur AAV artificiel comporte, dans le sens 5-prime à 3-prime :
une AAV-ITR 5-prime ;
l'ADN simple brin ;
une AAV-ITR interne ;
un complément inverse de l'ADN simple brin ; et
une AAV-ITR 3-prime.

23. Composition selon la revendication 14, dans laquelle le vecteur AAV artificiel a été traité thermiquement pendant au moins un cycle de chauffage et de refroidissement.

24. Composition selon la revendication 14 destinée à administrer un ADN simple brin à travers une barrière sang-cerveau en vue d'une expression dans le cerveau, la composition comportant :
un vecteur de virus adéno-associé artificiel (AAV) comportant l'ADN simple brin codant un agent biologiquement actif ; et
un composant pour administrer au moins l'ADN simple brin à travers la barrière sang-cerveau, dans lequel
a) l'agent biologiquement actif est codé par la SEQ ID N° : 2.

25. Composition selon la revendication 24, dans laquelle le vecteur AAV artificiel comporte une séquence choisie parmi le groupe constitué des SEQ ID N° : 8 à 11.

26. Composition selon la revendication 14, dans laquelle l'ADN simple brin codant l'agent biologiquement actif comporte une séquence codée par la SEQ ID N° : 2.

27. Composition selon la revendication 24, dans laquelle l'ADN simple brin code un petit ARN en épingle à cheveux.

28. Vecteur de virus adéno-associé artificiel (AAV) comportant, dans le sens 5-prime à 3-prime :
une AAV-ITR 5-prime ;
un ADN simple brin codant un agent biologiquement actif ;
une AAV-ITR interne ;
un complément inverse de l'ADN codant l'agent biologiquement actif ; et
une AAV-ITR 3-prime, dans lequel :
a) ledit ADN simple brin codent l'agent biologiquement actif est codé par la SEQ ID N° : 2 ; ou
b) ledit AAV artificiel est choisi parmi les SEQ ID N° : 8 à 11.

29. Vecteur selon la revendication 28, dans lequel le vecteur AAV artificiel comporte une séquence choisie parmi le groupe constitué des SEQ ID N° : 10 à 11.

30. Vecteur selon la revendication 28, dans lequel l'ADN simple brin codant l'agent biologiquement actif comporte une séquence de la SEQ ID N° : 2.

31. Vecteur selon la revendication 28, dans lequel l'ADN simple brin code un petit ARN en épingle à cheveux.

32. vecteur de virus adéno-associé artificiel (AAV) selon la revendication 28, dans lequel :
a) ledit ADN codant l'agent biologiquement actif est codé par la SEQ ID N° : 2 ; ou
b) ledit AAV artificiel est choisi parmi les SEQ ID N° : 10 à 11.

33. Vecteur selon la revendication 32, dans lequel le vecteur AAV artificiel a été traité thermiquement pendant au moins un cycle de chauffage et de refroidissement.

34. Vecteur selon la revendication 32, dans lequel l'ADN code un petit ARN en épingle à cheveux.

35. Composition selon l'une quelconque des revendications 14 à 27 destinée à une utilisation dans le traitement de la maladie d'Alzheimer.
